# EUROPÄISCHE PATENTSCHRIFT

(11) **EP 4 035 215 B1**
(45) Veröffentlichungstag und Bekanntmachung des Hinweises auf die Patenterteilung: **30.08.2023**
(21) Anmeldenummer: 20792522.3
(22) Anmeldetag: 24.09.2020
(51) Int. Cl.: H10K 85/60, H10K 30/30

(54) **VERBINDUNGEN MIT EINER FUROPYRROL- ODER EINER THIENOPYRROLGRUPPE, OPTOELEKTRONISCHE BAUELEMENTE MIT EINER SOLCHEN VERBINDUNG, UND VERWENDUNG EINER SOLCHEN VERBINDUNG IN OPTOELEKTRONISCHEN BAUELEMENTEN**
COMPOUNDS WITH A FUROPYRROLE OR A THIENOPYRROLE GROUP, OPTOELECTRONIC COMPONENT WITH SAID TYPE OF COMPOUND, AND USE OF SAID TYPE OF COMPOUND IN OPTOELECTRONIC COMPONENTS
COMPOSÉS POSSÉDANT UN GROUPE FUROPYROLLE OU UN GROUPE THIÉNOPYROLLE, COMPOSANT OPTOÉLECTRONIQUE COMPRENANT CE TYPE DE COMPOSÉ, ET UTILISATION DE CE TYPE DE COMPOSÉ DANS DES COMPOSANTS OPTOÉLECTRONIQUES

(30) Priorität: 24.09.2019 DE 102019125715
(43) Veröffentlichungstag der Anmeldung: 03.08.2022
(73) Patentinhaber: Heliatek GmbH, 01139 Dresden (DE)
(72) Erfinder: ANDERNACH, Rolf, 89077 Ulm (DE); GERDES, Olga, 89077 Ulm (DE); MATTERSTEIG, Gunter, 89077 Ulm (DE); RAMIREZ, Ivan, 01139 Dresden (DE); MIRLOUP, Antoine, 89077 Ulm (DE)
(86) Internationale Anmeldenummer: PCT/DE2020/100826
(87) Internationale Veröffentlichungsnummer: WO 2021/058065

(56) Entgegenhaltungen:
- WO-A1-2014/128277
- DE-A1-102013 101 712
- US-B2- 9 127 020

## Beschreibung

Die vorliegende Erfindung betrifft Verbindungen der allgemeinen Formel I, optoelektronische Bauelemente mit einer solchen Verbindung, sowie die Verwendung einer solchen Verbindung in optoelektronischen Bauelementen.

Für optoelektronische Bauelemente werden häufig Verschaltungen aus elektrisch leitfähigen Polymeren oder kleinen organischen Molekülen verwendet. Optoelektronische Bauelemente können beispielsweise Displays, Datenspeicher oder Transistoren sein, aber auch organisch optoelektronische Bauelemente, insbesondere organische photoaktive Bauelemente, beispielsweise Solarzellen und Photodetektoren, die eine photoaktive Schicht aufweisen, in der bei Einfall von elektromagnetischer Strahlung Ladungsträger, insbesondere gebundene Elektronen-Loch-Paare (Exzitonen), erzeugt werden.

Organische optoelektronische Bauelemente ermöglichen die Umwandlung von elektromagnetischer Strahlung unter Ausnutzung des photoelektrischen Effekts in elektrischen Strom. Für eine derartige Umwandlung von elektromagnetischer Strahlung werden Absorbermaterialien benötigt, die gute Absorptionseigenschaften zeigen. Weitere optoelektronische Bauelemente sind lichtemittierende elektrolumineszierende Bauelemente, die Licht aussenden, wenn sie von elektrischem Strom durchflossen werden. Optoelektronische Bauelemente umfassen mindestens zwei Elektroden, wobei eine Elektrode auf einem Substrat aufgebracht ist und die andere als Gegenelektrode fungiert, zwischen den Elektroden befindet sich mindestens ein Schichtsystem, wobei das Schichtsystem mindestens eine organische photoaktive Schicht aufweist. Zusätzlich können weitere Schichten, beispielsweise Ladungsträger-Transportschichten, zwischen den Elektroden angeordnet sein.

Ein aus dem Stand der Technik bekannter Aufbau einer organischen Solarzelle besteht in einer pin- oder nip-Struktur (Martin Pfeiffer, "Controlled doping of organic vacuum deposited dye layers: basics and applications", PhD thesis TU-Dresden, 1999, und WO2011/161108A1). Eine pin-Solarzelle besteht dabei aus einem Substrat mit daran anschließender meist transparenter Elektrode, p-Schicht(en), i-Schicht(en), n-Schicht(en) und einer Gegenelektrode, und eine nip-Solarzelle besteht aus einem Substrat mit daran anschließender meist transparenter Elektrode, n-Schicht(en), i-Schicht(en), p-Schicht(en) und einer Gegenelektrode.

Aus dem Stand der Technik sind zahlreiche Absorbermaterialien für organische Solarzellen bekannt.

WO2017114937A1 offenbart eine organische Verbindung, wobei sich die organische Verbindung durch eine hohe Absorption im kurzwelligen Spektralbereich des sichtbaren Lichts auszeichnet. Weiterhin wird die Verwendung dieser Verbindung für ein organisches elektronisches Bauelement sowie ein Verfahren zu Herstellung der Verbindung offenbart.

WO2017114938A1 offenbart ein organisches halbleitendes Material und dessen Verwendung in organischen Bauelementen, wobei das organische Material als funktionale Komponente in organischen elektronischen Bauelementen dienen kann, und zu einer verbesserten Absorption in organischen Solarzellen führt oder eine erhöhte Ladungsträger-Beweglichkeit aufweist.

WO2014128277A1 offenbart chemische Verbindungen der allgemeinen Formel A-D-A', wobei A und A' jeweils Akzeptoren sind und D ein unsymmetrischer Donorblock ist, und deren Verwendung in optoelektronischen Bauelementen.

Die im Stand der Technik offenbarten Absorbermaterialien sind zwar für photoaktive Schichten in organischen photovoltaischen Elementen, also organischen Solarzellen, geeignet, allerdings bedarf es einer Verbesserung der Absorptionseigenschaften der Absorbermaterialien, insbesondere um organische photovoltaische Elemente (OPVs) gegenüber herkömmlichen Solarzellen auf Siliziumbasis wettbewerbsfähig zu machen. Die Effizienz eines organischen photovoltaischen Elements hängt unter anderem vom dem Absorptionsverhalten der organischen Verbindungen, also den Absorbermaterialien, in der mindestens einen photoaktiven Schicht ab. Hierbei sind insbesondere in Tandem-, Triple-, oder Mehrfachzellen durch mehrere Absorbermaterialien, die in unterschiedlichen Wellenlängenbereichen absorbieren, eine hohe gesamte Absorption und eine Absorption in einem breiten Bereich des zur Verfügung stehenden Spektrums der elektromagnetischen Strahlung vorteilhaft, da damit Photonen verschiedener Wellenlängen für die Erzeugung von elektrischem Strom genutzt werden können. Die einzelnen Zellen der Tandem-, Triple-, oder Mehrfachzellen sollten eine Leerlaufspannung in der gleichen Größenordnung aufweisen, insbesondere in einem Bereich von 0,9 bis 1,1 V.

Nachteilig aus dem Stand der Technik sind insbesondere die Absorptionseigenschaften von Absorbermaterialien im blau/grün Bereich des sichtbaren Lichts, wobei insbesondere hohe AbsorptionsKoeffizienten, zum Aufbau von Solarzellen mit Leerlaufspannungen Uoc im Bereich von 0,9 V bis 1,1 V erforderlich sind. Bei Wellenlängen im Bereich von 500 nm - 600 nm ist die Absorption bekannter für organische photovoltaische Elemente geeigneter organischer Verbindungen nicht ausreichend. Dies ist insbesondere beim Aufbau von Tandem-, Triple- und Mehrfachzellen nachteilig, da die einzelnen Teilzellen nicht die gleichen Größenordnungen an Effizienz und Leerlaufspannung zeigen, also eine Teilzelle eine geringere Leerlaufspannung liefert als eine andere Teilzelle, wodurch eine Teilzelle nur einen geringeren Beitrag zum Wirkungsgrad des Bauelementes leistet.

Der Erfindung liegt daher die Aufgabe zugrunde, organische Verbindungen für den Einsatz in organischen optoelektronischen Bauelementen, ein optoelektronisches Bauelement mit mindestens einer solchen Verbindung, sowie die Verwendung solcher Verbindungen in einem optoelektronischen Bauelement bereitzustellen, wobei die genannten Nachteile nicht auftreten, und wobei die organischen Verbindungen insbesondere verbesserte Absorptionseigenschaften zeigen.

Die Aufgabe wird durch die Gegenstände der unabhängigen Ansprüche gelöst. Vorteilhafte Ausgestaltungen ergeben sich aus den Unteransprüchen.

Die Aufgabe wird insbesondere gelöst, indem eine Verbindung der allgemeinen Formel I bereitgestellt wird, 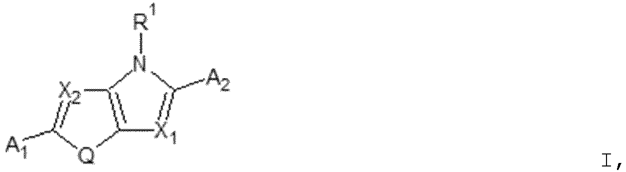 dadurch gekennzeichnet, dass
Q O oder S ist, bevorzugt ist Q S;
R1 ausgewählt ist aus der Gruppe bestehend aus Alkoxy, Alkyl, fluoriertem Alkyl, teilfluoriertem Alkyl, und Aryl;
X1 und X2 unabhängig voneinander N oder C-R4 sind; wobei
R4 ausgewählt ist aus der Gruppe bestehend aus H, Halogen, Alkoxy, verzweigtem oder linearem, zyklischem oder offenkettigem Alkyl, Alkenyl, und Aryl, wobei H jeweils substituiert sein kann;
A1 eine Gruppe mit der Formel Ia 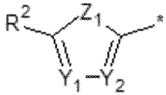 ist,
wobei * die Anknüpfung an die Verbindung der allgemeinen Formel I bezeichnet;
Z1 ausgewählt ist aus der Gruppe bestehend aus O, S, Se, und N-R5, wobei R5 ausgewählt ist aus der Gruppe bestehend aus H, Alkoxy, Alkyl, fluoriertem Alkyl, teilfluoriertem Alkyl, und Aryl;
Y1 N oder C-R6 ist, wobei R6 ausgewählt ist aus der Gruppe bestehend aus H, Halogen, Alkoxy, verzweigtem oder linearem, zyklischem oder offenkettigem Alkyl, Alkenyl, und Aryl, wobei H jeweils substituiert sein kann;
Y2 N oder C-R7 ist, wobei R7 ausgewählt ist aus der Gruppe bestehend aus H, Halogen, Alkoxy, verzweigtem oder linearem, zyklischem oder offenkettigem Alkyl, Alkenyl, und Aryl, wobei H jeweils substituiert sein kann; und wobei R6 und R7 miteinander in Form einer Ringstruktur homocyclisch oder heterocyclisch verbunden sein können; R2 ausgewählt ist aus der Gruppe bestehend aus H, Halogen, Alkoxy, Alkyl, fluoriertem Alkyl, teilfluoriertem Alkyl, verzweigtem oder linearem, zyklischem oder offenkettigem Alkyl, Amino, Aryl, Heteroaryl, Alkenyl, und einer elektronenziehenden Alkyl-Gruppe mit zumindest einer C=C-Doppelbindung, wobei H durch CN oder F substituiert sein kann;
A2 eine Gruppe mit der Formel Ib 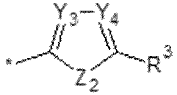 ist,
wobei * die Anknüpfung an die Verbindung der allgemeinen Formel I bezeichnet;
Z2 ausgewählt ist aus der Gruppe bestehend aus O, S, Se, und N-R8, wobei R8 ausgewählt ist aus der Gruppe bestehend aus H, Alkoxy, Alkyl, fluoriertem Alkyl, teilfluoriertem Alkyl, und Aryl;
Y3 N oder C-R9 ist, wobei R9 ausgewählt ist aus der Gruppe bestehend aus H, Halogen, Alkoxy, verzweigtem oder linearem, zyklischem oder offenkettigem Alkyl, Alkenyl, und Aryl, wobei H jeweils substituiert sein kann;
Y4 N oder C-R10 ist, wobei R10 ausgewählt ist aus der Gruppe bestehend aus H, Halogen, Alkoxy, verzweigtem oder linearem, zyklischem oder offenkettigem Alkyl, Alkenyl, und Aryl, wobei H jeweils substituiert sein kann; und wobei R9 und R10 miteinander in Form einer Ringstruktur homocyclisch oder heterocyclisch verbunden sein können; und
R3 ausgewählt ist aus der Gruppe bestehend aus H, Halogen, Alkoxy, Alkyl, fluoriertem Alkyl, teilfluoriertem Alkyl, verzweigtem oder linearem, zyklischem oder offenkettigem Alkyl, Amino, Aryl, Heteroaryl, Alkenyl, und einer elektronenziehenden Alkyl-Gruppe mit zumindest einer C=C-Doppelbindung, wobei H durch CN oder F substituiert sein kann.

In einer bevorzugten Ausführungsform der Erfindung ist Q S.

Die vorliegende Erfindung betrifft insbesondere A-D-A-Farbstoffe der Verbindung der allgemeinen Formel I mit einer zentralen Furopyrrol-Einheit oder einer zentralen Thienopyrrol-Einheit.

In einer bevorzugten Ausführungsform der Erfindung sind X1 und X2 H.

In einer bevorzugten Ausführungsform der Erfindung ist die Gruppe mit der Formel Ia und/oder die Gruppe mit der Formel Ib ein Furan oder ein Furan-Derivat.

In einer bevorzugten Ausführungsform der Erfindung ist die Gruppe mit der Formel Ia und/oder die Gruppe mit der Formel Ib ein Thiophen oder ein Thiophen-Derivat.

In einer bevorzugten Ausführungsform der Erfindung ist die Gruppe mit der Formel Ia und/oder die Gruppe mit der Formel Ib ein Pyrrol oder ein Pyrrol-Derivat.

In einer bevorzugten Ausführungsform der Erfindung ist die Gruppe mit der Formel Ia und/oder die Gruppe mit der Formel Ib ein Thiazol oder ein Thiazol-Derivat.

In einer bevorzugten Ausführungsform der Erfindung ist die Gruppe mit der Formel Ia und/oder die Gruppe mit der Formel Ib ein Thiadiazol oder ein Thiadiazol-Derivat.

In einer bevorzugten Ausführungsform der Erfindung ist die Gruppe mit der Formel Ia und/oder die Gruppe mit der Formel Ib ein Oxazol oder ein Oxazol-Derivat.

In einer bevorzugten Ausführungsform der Erfindung ist die Gruppe mit der Formel Ia und/oder die Gruppe mit der Formel Ib ein Imidazol oder ein Imidazol-Derivat.

In einer bevorzugten Ausführungsform der Erfindung ist die Gruppe mit der Formel Ia und/oder die Gruppe mit der Formel Ib ein Triazol oder ein Triazol-Derivat.

In einer bevorzugten Ausführungsform der Erfindung ist die Gruppe mit der Formel Ia und/oder die Gruppe mit der Formel Ib ein Oxadiazol oder ein Oxadiazol-Derivat.

In einer bevorzugten Ausführungsform der Erfindung sind mindestens zwei der Gruppen A1, A2, A3 oder A4 ein Furan, ein Thiophen, ein Pyrrol, ein Thiazol, ein Thiadiazol, ein Oxazol, ein Imidazol, ein Triazol, oder ein Oxadiazol, oder ein Derivat davon.

Unter einem Derivat wird im Zusammenhang mit der vorliegenden Erfindung insbesondere eine von einem Grundgerüst abgeleitete Verbindung oder ein Rest verstanden, wobei einzelne Atome oder Gruppen ausgehend von der ursprünglichen Verbindung modifiziert oder substituiert sind.

In einer bevorzugten Ausführungsform der Erfindung ist an der Furopyrrol-Einheit oder an der Thienopyrrol-Einheit, insbesondere an der Thienopyrrol-Einheit, eine Furan- und/oder ein Thiophen-Gruppe angeordnet, insbesondere eine Furan-Gruppe, und eine sterisch anspruchsvolle Gruppe am N des Pyrrols der Furopyrrol-Einheit oder der Thienopyrrol-Einheit, insbesondere iso-Propyl, iso-Butyl, sec-Butyl, tert-Butyl, iso-Pentyl oder Phenyl. Dadurch werden die die Eigenschaften der Absorbermaterialien in besonderer Weise verbessert.

In einer Bevorzugten Ausführungsform der Erfindung weist die zentrale Furopyrrol-Einheit oder die zentral Thienopyrrol-Einheit, insbesondere der Pyrrol-Ring der Thienopyrrol-Einheit, kein weiteres kondensiertes aromatisches System auf.

In einer bevorzugten Ausführungsform der Erfindung sind R5 und R6 H.

In einer alternativ bevorzugten Ausführungsform der Erfindung weist die Verbindung der allgemeinen Formel I einen jeweils zusammen durch R5 und R6 und/oder R9 und R10 gebildeten homocyclischen Fünf-Ring oder Sechs-Ring, oder einen heterocyclischen Fünf-Ring oder Sechs-Ring mit mindestens einem Heteroatom ausgewählt aus der Gruppe bestehend aus S, O und N auf, wobei der Fünf-Ring oder der Sechs-Ring an weiteren Positionen substituiert sein kann.

Unter einem Absorbermaterial, also einem Absorber, wird insbesondere eine Verbindung verstanden, die Licht im Wellenlängenbereich von >400 nm absorbiert. Unter einer Absorberschicht wird dementsprechend insbesondere eine Schicht in einem optoelektronischen Bauelement verstanden, die mindestens ein Absorbermaterial aufweist.

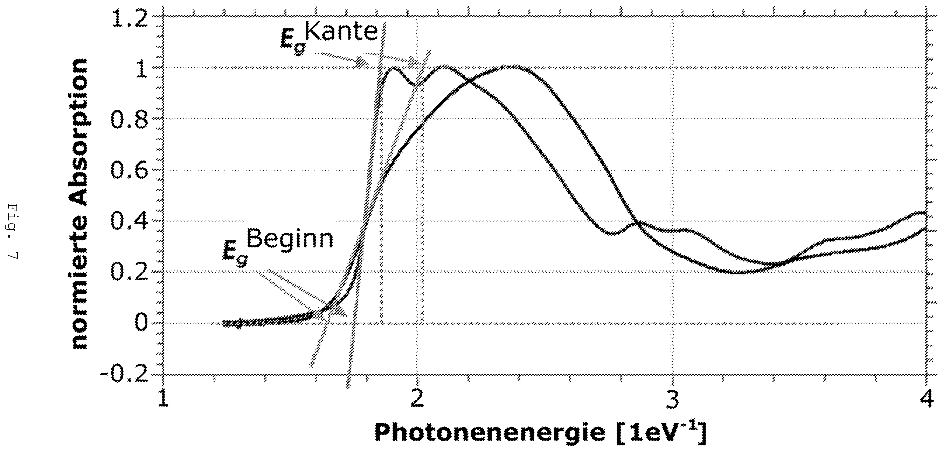

Unter einem steilen Verlauf des Absorptionsspektrums, insbesondere einem steilen Verlauf im bathochromen Bereich des Absorptionsspektrums, wird insbesondere eine Steilheit an einem Wendepunkt des Absorptionsspektrums verstanden. Die Steilheit des Verlaufs des Absorptionsspektrums wird dabei über die Steigung der Tangente an dem Wendepunkt bestimmt, der zwischen dem Beginn der Absorption und der ersten Kante der Absorption liegt (Wang et al., "Optical Gaps of Organic Solar Cells as a Reference for Comparing Voltage Losses", Adv. Energy Mater., 2018, 1801352), vergleiche Fig. 7.

In einer bevorzugten Ausführungsform der Erfindung weißen erfindungsgemäße Verbindungen im Absorptionsspektrum eine Steilheit von mindestens 5,6 auf, bevorzugt von mindestens 5,8, bevorzugt von mindestens 6, bevorzugt von mindestens 6,5, bevorzugt von mindestens 7, bevorzugt von mindestens 7,5, oder bevorzugt von mindestens 8.

Unter einer Substitution wird insbesondere der Austausch von H durch einen Substituenten verstanden. Unter einem Substituenten werden insbesondere alle Atome und Atomgruppen außer H verstanden, bevorzugt ein Halogen, eine Alkyl-Gruppe, dabei kann die Alkyl-Gruppe linear oder verzweigt sein, eine Amino-Gruppe, eine CN-Gruppe, eine Alkenyl-Gruppe, eine Alkinyl-Gruppe, eine AlkoxyGruppe, eine Thioalkoxy-Gruppe, eine Aryl-Gruppe, oder eine Heteroaryl-Gruppe. Unter einem Halogen wird insbesondere F, Cl oder Br verstanden, bevorzugt F.

Unter einem Heteroatom, insbesondere einem Heteroatom wird insbesondere ein Atom ausgewählt aus der Gruppe bestehend aus O, S, Se, Si, B, N oder P verstanden, bevorzugt ausgewählt aus der Gruppe bestehend aus O, S, Se oder N, insbesondere bevorzugt O oder S.

In einer bevorzugten Ausführungsform der Erfindung ist kein C einer Alkyl-Gruppe durch ein Heteroatom ersetzt.

Die erfindungsgemäßen Verbindungen betreffen insbesondere sogenannte kleine Moleküle. Unter kleinen Molekülen werden insbesondere nichtpolymere organische Moleküle mit monodispersen molaren Massen zwischen 100 und 2000 g/mol verstanden, die unter Normaldruck (Luftdruck der uns umgebenden Atmosphäre) und bei Raumtemperatur in fester Phase vorliegen. Insbesondere sind die kleinen Moleküle photoaktiv, wobei unter photoaktiv verstanden wird, dass die Moleküle unter Lichteintrag ihren Ladungszustand und/oder ihren Polarisierungszustand ändern. Die photoaktiven Moleküle zeigen insbesondere eine Absorption elektromagnetischer Strahlung in einem bestimmten Wellenlängenbereich, wobei absorbierte elektromagnetische Strahlung, also Photonen, in Exzitonen umgewandelt werden.

Die erfindungsgemäßen Verbindungen zeigen insbesondere einen hohen Absorptionskoeffizienten zumindest in einem bestimmten Wellenlängenbereich. Die erfindungsgemäßen Verbindungen werden insbesondere in einem Donor-Akzeptor-System mit Heterojunction eingesetzt.

In einer bevorzugten Ausführungsform der Erfindung sind Y1, Y2, Y3, und Y4 jeweils unabhängig voneinander ausgewählt aus der Gruppe bestehend aus N, CH, CF, C-CH₃, C-CF₃, C-C₂H₅, C-C₃H₈, C-OCH₃, C-OC₂H₅, C-SCH₃, C-SC₂H₅.

In einer bevorzugten Ausführungsform der Erfindung sind die zyklischen oder lineare Alkyl-Gruppen der erfindungsgemäßen Verbindungen linear oder verzweigt, bevorzugt sind die Alkyl-Gruppen lineare C1-C5-Alkyl-Gruppen.

In einer bevorzugten Ausführungsform der Erfindung ist zumindest eine der Positionen Y1, Y2, Y3 und Y4 ein N, bevorzugt ist mindestens eine der Positionen Y1 und Y2 ein N, und mindestens eine der Positionen Y3 und Y4 ein N. In einer besonders bevorzugten Ausführungsform der Erfindung sind die Positionen Y1, Y2, Y3, und Y4 jeweils ein N.

In einer bevorzugten Ausführungsform der Erfindung sind die Positionen Y1, Y2, Y3, und Y4 jeweils CH.

In einer bevorzugten Ausführungsform der Erfindung weist die Verbindung der allgemeinen Formel I an der Position Z1 der Formel Ia ein O und an der Position Z2 der Formel Ib ein O auf. Dadurch sind insbesondere die Absorptionseigenschaften günstiger als bei vergleichbaren Verbindungen mit einem anderen Atom an diesen Positionen.

Die erfindungsgemäßen Verbindungen weisen Vorteile im Vergleich zum Stand der Technik auf. Vorteilhafterweise weisen die erfindungsgemäßen Verbindungen ein überraschend gutes Absorptionsverhalten in einem vergleichsweise breiten Spektralbereich des sichtbaren Lichts auf, insbesondere konnte eine überraschend hohe Absorption im Spektralbereich von ca. 500 nm bis 600 nm festgestellt werden. Vorteilhafterweise werden Absorbermaterialien mit einer verbesserten Absorption in einem Wellenlängenbereich des blau/grün Bereichs des sichtbaren Lichts bereitgestellt. Vorteilhafterweise zeigen erfindungsgemäße Verbindungen einen besonders steilen Verlauf der Absorption im bathochromen Bereich. Vorteilhafterweise verfügen die erfindungsgemäßen Verbindungen über eine hinreichende thermische, chemische und elektrochemische Stabilität, um den Anforderungen gerecht zu werden, die bei der Fertigung und im Betrieb von photoaktiven organischen elektronischen Bauelementen üblicherweise an sie gestellt werden, insbesondere sind diese Verbindungen gut im Vakuum verdampfbar. Vorteilhafterweise ermöglichen die erfindungsgemäßen Verbindungen den Aufbau von Solarzellen mit einer Leerlaufspannung Uoc im Bereich von 0,9 bis 1,1 V, insbesondere im Bereich von 0,95 V bis 1,05 V.

Gemäß einer Weiterbildung der Erfindung ist vorgesehen, dass
R1 ausgewählt ist aus der Gruppe bestehend aus Ethyl, Propyl, Butyl, verzweigtem Alkyl, und Aryl, bevorzugt aus iso-Propyl, iso-Butyl, sec-Butyl, iso-Pentyl, Tert-Butyl und Phenyl; und
wobei Z1 und Z2 jeweils unabhängig voneinander O oder S sind.

In einer besonders bevorzugten Ausführungsform ist Z2 O. Dadurch verwirklichen sich die vorteilhaften Effekte der vorliegenden Erfindung in besonderer Weise.

Gemäß einer Weiterbildung der Erfindung ist vorgesehen, dass die Verbindung eine Verbindung der allgemeinen Formel II ist, 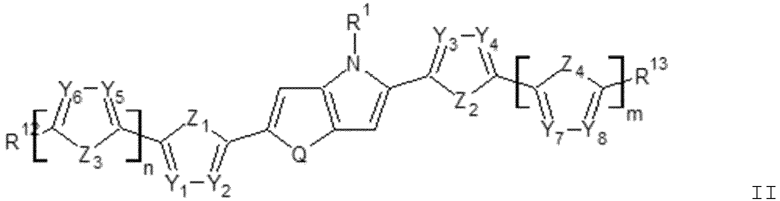
wobei n 0, 1 oder 2 ist und m 0, 1 oder 2 ist;
Q O oder S ist, bevorzugt ist Q S;
Z3 und Z4 jeweils unabhängig voneinander ausgewählt sind aus der Gruppe bestehend aus O, S, Se, und N-R11, wobei R11 ausgewählt ist aus der Gruppe bestehend aus H, Alkoxy, Alkyl, fluoriertem Alkyl, teilfluoriertem Alkyl, und Aryl;
R12 ausgewählt ist aus der Gruppe bestehend aus H, Alkoxy, Alkyl, fluoriertem Alkyl, teilfluoriertem Alkyl, Alkenyl, und einer elektronenziehenden Alkyl-Gruppe mit zumindest einer C=C-Doppelbindung, wobei H durch CN oder F substituiert sein kann;
R13 ausgewählt ist aus der Gruppe bestehend aus H, Alkoxy, Alkyl, fluoriertem Alkyl, teilfluoriertem Alkyl, Alkenyl, und einer elektronenziehenden Alkyl-Gruppe mit zumindest einer C=C-Doppelbindung, wobei H durch CN oder F substituiert sein kann;
Y5 N oder C-R14 ist, wobei R14 ausgewählt ist aus der Gruppe bestehend aus H, Halogen, Alkoxy, Alkyl, und Alkenyl, wobei H jeweils substituiert sein kann, wobei Y5 bevorzugt N, CH oder CF ist;
Y6 N oder C-R15 ist, wobei R15 ausgewählt ist aus der Gruppe bestehend aus H, Halogen, Alkoxy, Alkyl, und Alkenyl, wobei H jeweils substituiert sein kann, wobei Y6 bevorzugt N, CH oder CF ist;
Y7 N oder C-R16 ist, wobei R16 ausgewählt ist aus der Gruppe bestehend aus H, Halogen, Alkoxy, Alkyl, und Alkenyl, wobei H jeweils substituiert sein kann, wobei Y7 bevorzugt N, CH oder CF ist;
Y8 N oder C-R17 ist, wobei R17 ausgewählt ist aus der Gruppe bestehend aus H, Halogen, Alkoxy, Alkyl, und Alkenyl, wobei H jeweils substituiert sein kann, wobei Y8 bevorzugt N, CH oder CF ist; und
wobei jeweils zusammen R14 und R15, und/oder R16 und R17 einen homocyclischen Fünf-Ring oder Sechs-Ring, oder einen heterocyclischen Fünf-Ring oder Sechs-Ring mit mindestens einem Heteroatom ausgewählt aus der Gruppe bestehend aus S, O und N bilden können.

In einer bevorzugten Ausführungsform der Erfindung sind Z3 und Z4 unabhängig voneinander O oder S.

In einer bevorzugten Ausführungsform der Erfindung weist die Verbindung der allgemeinen Formel II an der Position Z1 ein O und an der Position Z2 ein O auf, und an der Position Z3 ein O und/oder an der Position Z4 ein O auf.

Gemäß einer Weiterbildung der Erfindung ist vorgesehen, dass A1 eine Gruppe mit der Formel IIa 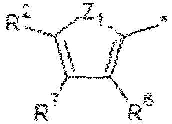 ist,
wobei R6 ausgewählt ist aus der Gruppe bestehend aus H, Halogen, Alkoxy, verzweigtem oder linearem, zyklischem oder offenkettigem Alkyl, Alkenyl, und Aryl, wobei H jeweils substituiert sein kann;
R7 ausgewählt ist aus der Gruppe bestehend aus H, Halogen, Alkoxy, verzweigtem oder linearem, zyklischem oder offenkettigem Alkyl, Alkenyl, und Aryl, wobei H jeweils substituiert sein kann; und wobei R6 und R7 zusammen einen homocyclischen Fünf-Ring oder Sechs-Ring, oder einen heterocyclischen Fünf-Ring oder Sechs-Ring mit mindestens einem Heteroatom ausgewählt aus der Gruppe bestehend aus S, O und N bilden können;
und A2 eine Gruppe mit der Formel IIb 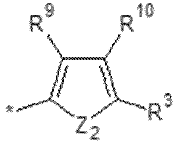 ist,
wobei R9 ausgewählt ist aus der Gruppe bestehend aus H, Halogen, Alkoxy, verzweigtem oder linearem, zyklischem oder offenkettigem Alkyl, Alkenyl, und Aryl, wobei H jeweils substituiert sein kann; R10 ausgewählt ist aus der Gruppe bestehend aus H, Halogen, Alkoxy, verzweigtem oder linearem, zyklischem oder offenkettigem Alkyl, Alkenyl, und Aryl, wobei H jeweils substituiert sein kann; und wobei R9 und R10 zusammen einen homocyclischen Fünf-Ring oder Sechs-Ring, oder einen heterocyclischen Fünf-Ring oder Sechs-Ring mit mindestens einem Heteroatom ausgewählt aus der Gruppe bestehend aus S, O und N bilden können.

In einer besonders bevorzugten Ausführungsform der Erfindung ist die Verbindung eine Verbindung der allgemeinen Formel XIX 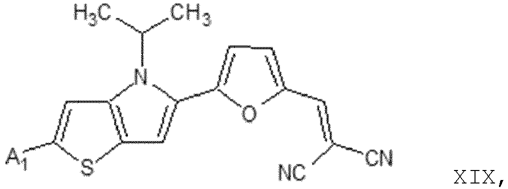 dadurch gekennzeichnet, dass
A1 eine Gruppe mit der Formel Ia 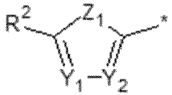 ist,
wobei * die Anknüpfung an die Verbindung der allgemeinen Formel I bezeichnet;
Z1 ausgewählt ist aus der Gruppe bestehend aus O, S, Se, und N-R5, wobei R5 ausgewählt ist aus der Gruppe bestehend aus H, Alkoxy, Alkyl, fluoriertem Alkyl, teilfluoriertem Alkyl, und Aryl;
Y1 N oder C-R6 ist, wobei R6 ausgewählt ist aus der Gruppe bestehend aus H, Halogen, Alkoxy, verzweigtem oder linearem, zyklischem oder offenkettigem Alkyl, Alkenyl, und Aryl, wobei H jeweils substituiert sein kann;
Y2 N oder C-R7 ist, wobei R7 ausgewählt ist aus der Gruppe bestehend aus H, Halogen, Alkoxy, verzweigtem oder linearem, zyklischem oder offenkettigem Alkyl, Alkenyl, und Aryl, wobei H jeweils substituiert sein kann; und wobei R6 und R7 miteinander in Form einer Ringstruktur homocyclisch oder heterocyclisch verbunden sein können; R2 ausgewählt ist aus der Gruppe bestehend aus H, Halogen, Alkoxy, Alkyl, fluoriertem Alkyl, teilfluoriertem Alkyl, verzweigtem oder linearem, zyklischem oder offenkettigem Alkyl, Amino, Aryl, Heteroaryl, Alkenyl, und einer elektronenziehenden Alkyl-Gruppe mit zumindest einer C=C-Doppelbindung, wobei H durch CN oder F substituiert sein kann.

In einer bevorzugten Ausführungsform der Erfindung sind die Positionen Y1 und Y2 der Verbindung der allgemeinen Formel XIX jeweils CH.

In einer bevorzugten Ausführungsform der Erfindung weist die Verbindung der allgemeinen Formel XIX an der Position Z1 der Formel Ia ein O auf.

In einer bevorzugten Ausführungsform der Erfindung ist zumindest eine der Positionen Y1 oder Y2 der Verbindung der allgemeinen Formel XIX ein N, bevorzugt sind die Positionen Y1 und Y2 ein N.

Gemäß einer Weiterbildung der Erfindung ist vorgesehen, dass die Verbindung eine Verbindung der allgemeinen Formel III, IV und/oder V ist, 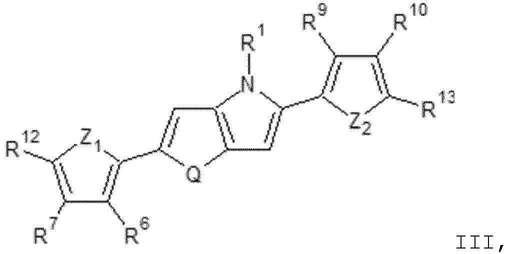 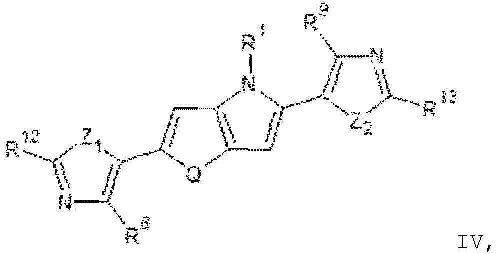 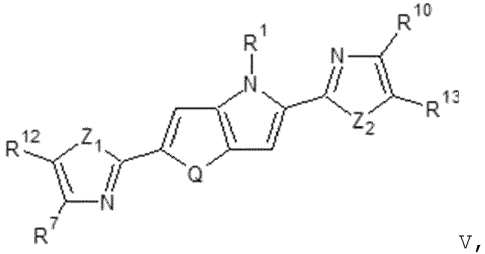
wobei Q O oder S ist, bevorzugt ist Q S;
Z1 und Z2 jeweils unabhängig voneinander O oder S sind, bevorzugt ist Z2 O;
R6 ausgewählt ist aus der Gruppe bestehend aus H, Halogen, Alkoxy, Alkyl, und Alkenyl, wobei H jeweils substituiert sein kann;
R7 ausgewählt ist aus der Gruppe bestehend aus H, Halogen, Alkoxy, Alkyl, und Alkenyl, wobei H jeweils substituiert sein kann; und
wobei R6 und R7 zusammen einen homocyclischen Fünf-Ring oder Sechs-Ring, oder einen heterocyclischen Fünf-Ring oder Sechs-Ring mit mindestens einem Heteroatom ausgewählt aus der Gruppe bestehend aus S, O und N bilden können;
R9 ausgewählt ist aus der Gruppe bestehend aus H, Halogen, Alkoxy, Alkyl, und Alkenyl, wobei H jeweils substituiert sein kann;
R10 ausgewählt ist aus der Gruppe bestehend aus H, Halogen, Alkoxy, Alkyl, und Alkenyl, wobei H jeweils substituiert sein kann; und
wobei R9 und R10 zusammen einen homocyclischen Fünf-Ring oder Sechs-Ring, oder einen heterocyclischen Fünf-Ring oder Sechs-Ring mit mindestens einem Heteroatom ausgewählt aus der Gruppe bestehend aus S, O und N bilden können;
R12 ausgewählt ist aus der Gruppe bestehend aus H, Alkoxy, Alkyl, fluoriertem Alkyl, teilfluoriertem Alkyl, Alkenyl, und einer elektronenziehenden Alkyl-Gruppe mit zumindest einer C=C-Doppelbindung, wobei H durch CN oder F substituiert sein kann; und R13 ausgewählt ist aus der Gruppe bestehend aus H, Alkoxy, Alkyl, fluoriertem Alkyl, teilfluoriertem Alkyl, Alkenyl, und einer elektronenziehenden Alkyl-Gruppe mit zumindest einer C=C-Doppelbindung, wobei H durch CN oder F substituiert sein kann.

In einer bevorzugten Ausführungsform der Erfindung ist vorgesehen, dass die Verbindung eine Verbindung ausgewählt aus den allgemeinen Formeln 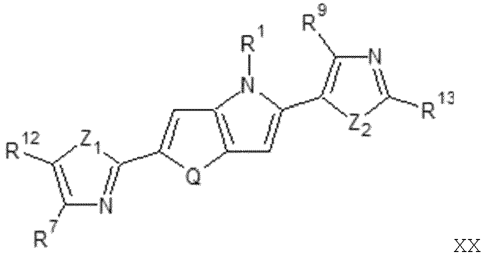 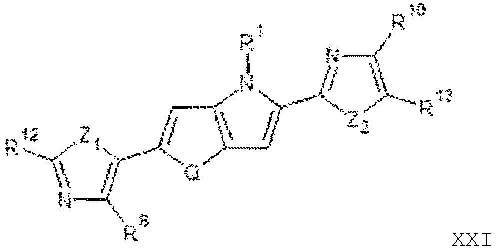 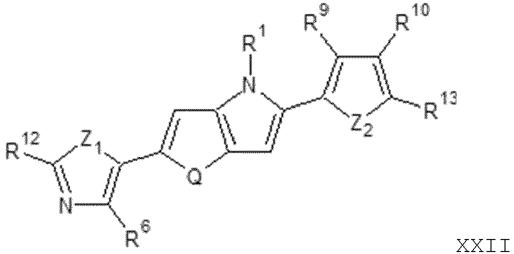 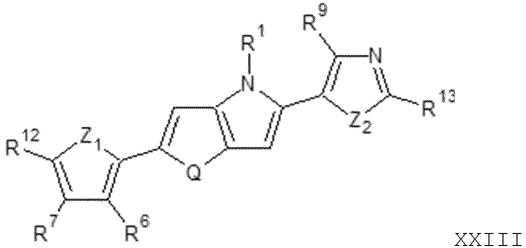 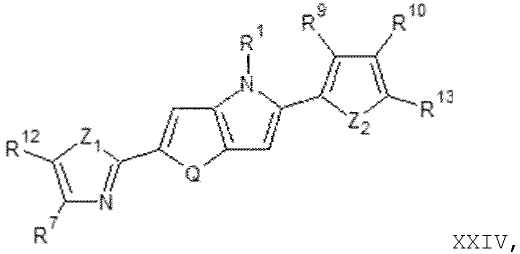 und 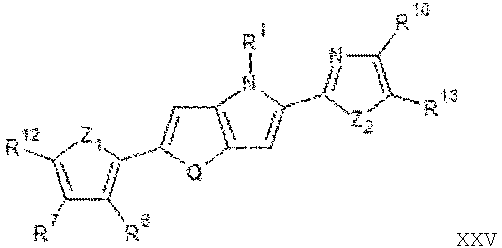 ist,
wobei Q O oder S ist, bevorzugt ist Q S;
Z1 und Z2 jeweils unabhängig voneinander O oder S sind, bevorzugt ist Z2 O;
R6 ausgewählt ist aus der Gruppe bestehend aus H, Halogen, Alkoxy, Alkyl, und Alkenyl, wobei H jeweils substituiert sein kann;
R7 ausgewählt ist aus der Gruppe bestehend aus H, Halogen, Alkoxy, Alkyl, und Alkenyl, wobei H jeweils substituiert sein kann; und
wobei R6 und R7 zusammen einen homocyclischen Fünf-Ring oder Sechs-Ring, oder einen heterocyclischen Fünf-Ring oder Sechs-Ring mit mindestens einem Heteroatom ausgewählt aus der Gruppe bestehend aus S, O und N bilden können;
R9 ausgewählt ist aus der Gruppe bestehend aus H, Halogen, Alkoxy, Alkyl, und Alkenyl, wobei H jeweils substituiert sein kann;
R10 ausgewählt ist aus der Gruppe bestehend aus H, Halogen, Alkoxy, Alkyl, und Alkenyl, wobei H jeweils substituiert sein kann; und wobei R9 und R10 zusammen einen homocyclischen Fünf-Ring oder Sechs-Ring, oder einen heterocyclischen Fünf-Ring oder Sechs-Ring mit mindestens einem Heteroatom ausgewählt aus der Gruppe bestehend aus S, O und N bilden können;
R12 ausgewählt ist aus der Gruppe bestehend aus H, Alkoxy, Alkyl, fluoriertem Alkyl, teilfluoriertem Alkyl, Alkenyl, und einer elektronenziehenden Alkyl-Gruppe mit zumindest einer C=C-Doppelbindung, wobei H durch CN oder F substituiert sein kann; und
R13 ausgewählt ist aus der Gruppe bestehend aus H, Alkoxy, Alkyl, fluoriertem Alkyl, teilfluoriertem Alkyl, Alkenyl, und einer elektronenziehenden Alkyl-Gruppe mit zumindest einer C=C-Doppelbindung, wobei H durch CN oder F substituiert sein kann.

In einer bevorzugten Ausführungsform der Erfindung ist vorgesehen, dass die Verbindung eine Verbindung der allgemeinen Formel VII und VIII ist, 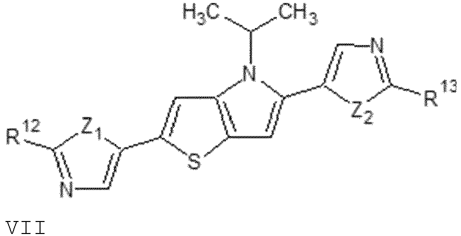 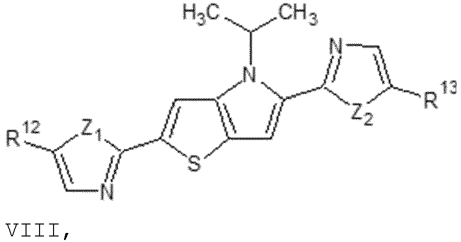
wobei Z1 und Z2 jeweils unabhängig voneinander O oder S sind,
R12 ausgewählt ist aus der Gruppe bestehend aus H, Alkoxy, Alkyl, fluoriertem Alkyl, teilfluoriertem Alkyl, Alkenyl, und einer elektronenziehenden Alkyl-Gruppe mit zumindest einer C=C-Doppelbindung, wobei H durch CN oder F substituiert sein kann; und
R13 ausgewählt ist aus der Gruppe bestehend aus H, Alkoxy, Alkyl, fluoriertem Alkyl, teilfluoriertem Alkyl, Alkenyl, und einer elektronenziehenden Alkyl-Gruppe mit zumindest einer C=C-Doppelbindung, wobei H durch CN oder F substituiert sein kann.

Gemäß einer Weiterbildung der Erfindung ist vorgesehen, dass R2 und R3, oder R12 und R13 jeweils unabhängig voneinander 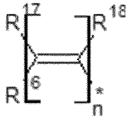 sind,
wobei n 1, 2 oder 3 ist, wobei * die Anknüpfung der Gruppe R2, R3, R12 und/oder R13 bezeichnet;
R16, R17 und R18 jeweils unabhängig voneinander ausgewählt sind aus der Gruppe bestehend aus H, Halogen, CN, COO-Alkyl, Alkyl und Alkenyl, mit der Bedingung, dass R16 und R17 nicht beide H sind, wobei bevorzugt R16 und R17 CN sind. Dadurch verwirklichen sich die vorteilhaften Effekte der vorliegenden Erfindung in besonderer Weise.

In einer bevorzugten Ausführungsform der Erfindung ist R2 und/oder R3, oder R12 und/oder R13 jeweils unabhängig voneinander 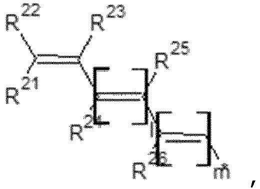
wobei m 0, 1 oder 2 ist, und 1 0, 1 oder 2 ist; wobei * die Anknüpfung der Gruppe R2, R3, R12 und/oder R13 bezeichnet;
R21, R22, R23, R24, R25 und R26 jeweils unabhängig voneinander ausgewählt sind aus der Gruppe bestehend aus H, Halogen, CN, COO-Alkyl, und Alkenyl, mit der Bedingung, dass R21 und R22 nicht beide H sind, wobei bevorzugt R21 und R22 CN sind. Vorzugsweise kann durch eine große Anzahl an CN-Gruppen eine besonders gute Akzeptor-Wirkung der Gruppen R2 und/oder R3, oder R12 und/oder R13 erreicht werden.

Gemäß einer Weiterbildung der Erfindung ist vorgesehen, dass
R2 und R3, oder R12 und R13 jeweils unabhängig voneinander ausgewählt sind aus der Gruppe bestehend aus: 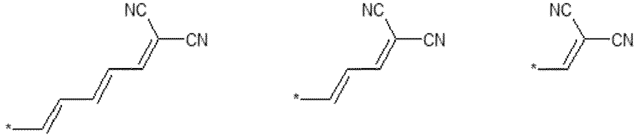 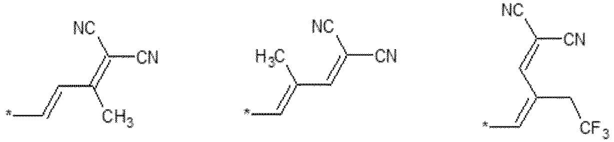 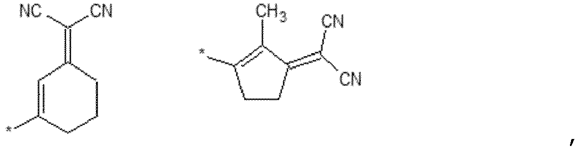
wobei * die Anknüpfung der Gruppe R2, R3, R12 und/oder R13 bezeichnet, wobei CN durch F ersetzt sein kann, und wobei bevorzugt R2 und R3, oder R12 und R13 gleich sind.

Gemäß einer Weiterbildung der Erfindung ist vorgesehen, dass R6 und R7 H sind, R9 und R10 H sind, und Z2 O ist; wobei bevorzugt Z1 und Z2 O sind. Dadurch verwirklichen sich die vorteilhaften Effekte der vorliegenden Erfindung in besonderer Weise.

In einer bevorzugten Ausführungsform der Erfindung ist R4 H, sind R6 und R7 H und/oder sind R9 und R10 H, und sind Z1 und Z2 O.

Gemäß einer Weiterbildung der Erfindung ist vorgesehen, dass die Verbindung ausgewählt ist aus der Gruppe bestehend aus:

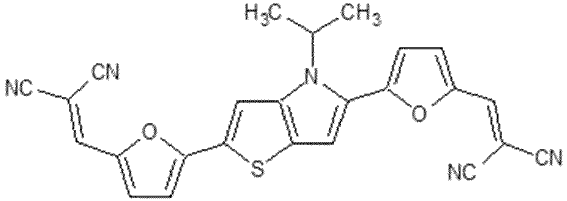

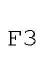

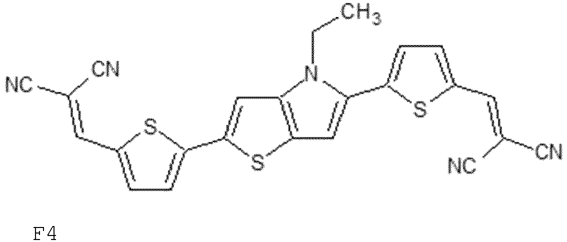

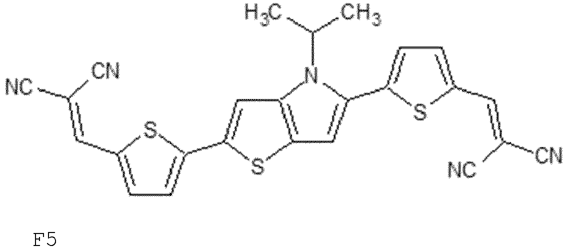

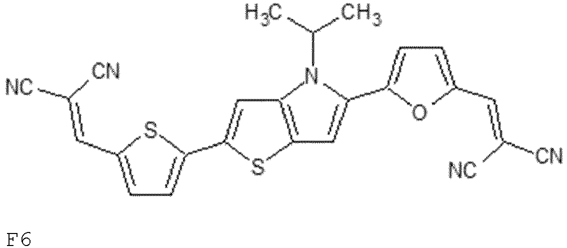

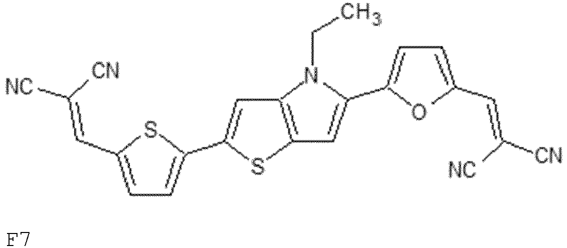

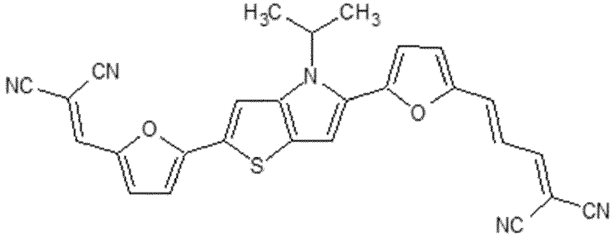

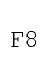

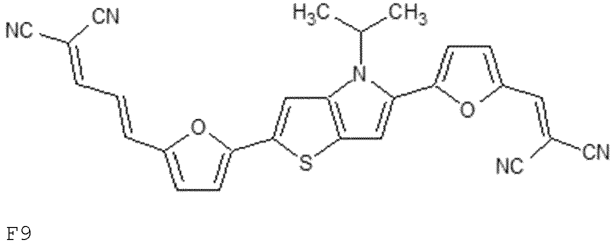

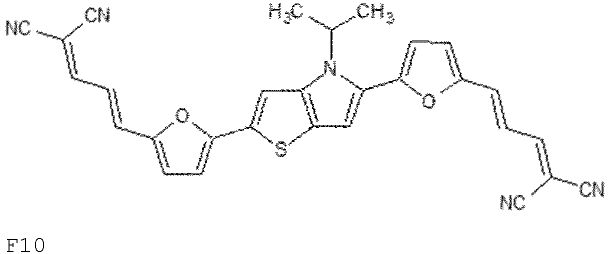

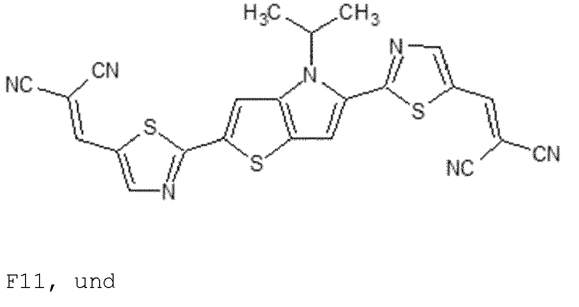

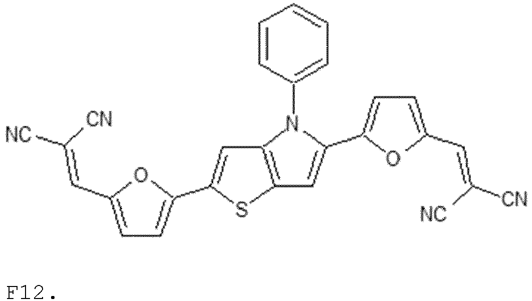

In einer bevorzugten Ausführungsform der Erfindung weisen R2 und/oder R3, oder R12 und/oder R13 mindestens zwei C=C Doppelbindungen auf.

Gemäß einer Weiterbildung der Erfindung ist vorgesehen, dass die Gruppe A1 gleich der Gruppe A2 ist.

In einer besonders bevorzugten Ausführungsform der Erfindung ist die Verbindung die Verbindung F3 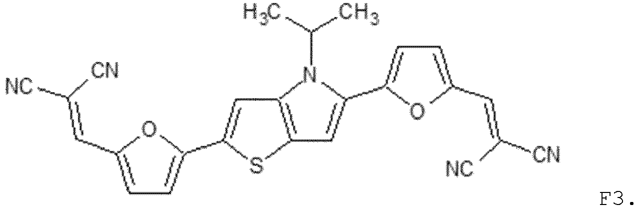 Hierbei verwirklichen sich die vorteilhaften Absorptionseigenschaften in besonderem Maße.

In einer besonders bevorzugten Ausführungsform der Erfindung ist die Verbindung die Verbindung F6 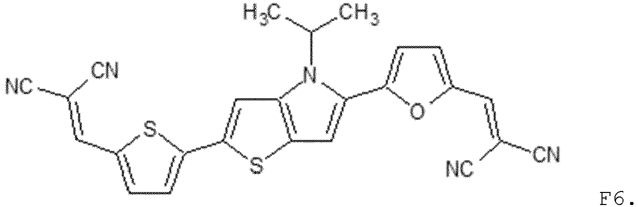 Hierbei verwirklichen sich die vorteilhaften Absorptionseigenschaften in besonderem Maße.

In einer besonders bevorzugten Ausführungsform der Erfindung ist die Verbindung die Verbindung F12 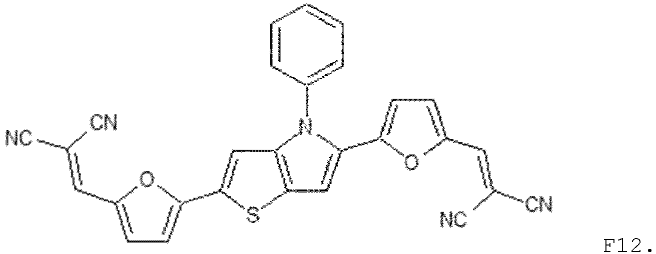 Hierbei verwirklichen sich die vorteilhaften Absorptionseigenschaften in besonderem Maße.

Aus dem Stand der Technik sind organische Einfach- oder Tandemzellen bekannt. DE102004014046A1 offenbart ein photoaktives Bauelement, insbesondere eine Solarzelle, bestehend aus organischen Schichten aus einer oder mehreren aufeinander gestapelten pi-, ni- und/oder pin-Dioden. WO2011161108A1 offenbart ein photoaktives Bauelement mit einer Elektrode und einer Gegenelektrode, wobei zwischen den Elektroden zumindest ein organisches Schichtsystem angeordnet ist, mit mindestens zwei photoaktiven Schichtsystemen und zwischen den photoaktiven Schichtsystemen zumindest zwei verschiedene Transportschichtsysteme des gleichen Ladungsträgertyps. Dabei ist das eine Transportschichtsystem energetisch an eines der beiden photoaktiven Schichtsysteme angepasst, und das andere Transportschichtsystem transparent ausgeführt.

Die Aufgabe der vorliegenden Erfindung wird auch gelöst, indem ein optoelektronisches Bauelement mit mindestens einer erfindungsgemäßen Verbindung bereitgestellt wird, insbesondere nach einem der zuvor beschriebenen Ausführungsbeispiele. Dabei ergeben sich für das optoelektronische Bauelement insbesondere die Vorteile, die bereits in Zusammenhang mit der erfindungsgemäßen Verbindung erläutert wurden. Das optoelektronische Bauelement umfasst dabei eine erste Elektrode, eine zweite Elektrode und ein Schichtsystem, wobei das Schichtsystem zwischen der ersten Elektrode und der zweiten Elektrode angeordnet ist, dadurch gekennzeichnet, dass mindestens eine Schicht des Schichtsystems mindestens eine erfindungsgemäße Verbindung aufweist.

Unter einem organischen optoelektronischen Bauelement wird insbesondere ein Bauelement verstanden, das organische leitende oder halbleitende Materialien aufweist, insbesondere ein Transistor, ein lichtemittierendes organisches Bauelement, ein organisches photovoltaisches Element (OPV), insbesondere eine organische Solarzelle, oder ein Photodetektor. Organische photovoltaische Zellen mit mindestens einer erfindungsgemäßen Verbindung ermöglichen eine besonders effiziente Nutzung des Spektrums des sichtbaren Lichts.

Unter einem organischen photovoltaischen Element (OPV) wird insbesondere ein photovoltaisches Element, insbesondere eine Solarzelle, mit mindestens einer organischen photoaktiven Schicht verstanden, wobei die organische photoaktive Schicht mindestens eine erfindungsgemäße Verbindung aufweist. Ein organisches photovoltaisches Element ermöglicht es elektromagnetische Strahlung, insbesondere im Wellenlängenbereich des sichtbaren Lichts, unter Ausnutzung des photoelektrischen Effekts in elektrischen Strom umzuwandeln.

Gemäß einer Weiterbildung der Erfindung ist vorgesehen, dass das optoelektronische Bauelement ein organisches optoelektronisches Bauelement ist, bevorzugt eine organische Solarzelle, ein OFET, eine OLED oder ein organischer Photodetektor.

Gemäß einer Weiterbildung der Erfindung ist vorgesehen, dass das Schichtsystem mindestens eine photoaktive Schicht aufweist, bevorzugt eine Absorberschicht, wobei die mindestens eine photoaktive Schicht die mindestens eine erfindungsgemäße Verbindung aufweist.

Das organische elektronische Bauelement weist dabei insbesondere eine Elektrode und eine Gegenelektrode auf, wobei zwischen den Elektroden eine organische photoaktive Schicht angeordnet ist. Bei der organischen photoaktiven Schicht handelt es sich insbesondere um eine photoaktive Schicht, bei der durch Strahlung von sichtbarem Licht, UV-Strahlung und/oder IR-Strahlung Exzitonen (Elektron-Loch-Paare) gebildet werden. Die organischen Materialien werden dabei in Form dünner Filme oder kleiner Volumen auf die Folien aufgedruckt, aufgeklebt, gecoated, aufgedampft oder anderweitig aufgebracht. Für die Herstellung der dünnen Schichten kommen ebenso alle Verfahren in Betracht, die auch für Elektronik auf Glas, keramischen oder halbleitenden Trägern verwendet werden. Die organische photoaktive Schicht weist dabei eine für das optoelektronische Bauelement wichtige Funktion auf, insbesondere eine ladungsträgertransportierende Funktion, wie der Transport von Löchern (p-leitend) oder der Transport von Elektronen (n-leitend).

Gemäß einer Weiterbildung der Erfindung ist vorgesehen, dass das Schichtsystem mindestens zwei photoaktive Schichten, bevorzugt mindestens drei photoaktive Schichten, oder bevorzugt mindestens vier photoaktive Schichten aufweist, wobei die photoaktiven Schichten bevorzugt Absorberschichten sind. In einer bevorzugten Ausführungsform der Erfindung ist die mindestens eine photoaktive Schicht zwischen der ersten Elektrode und der zweiten Elektrode angeordnet.

In einer bevorzugten Ausführungsform der Erfindung weist die organische Solarzelle eine photoaktive Schicht auf, die wenigstens ein organisches Donor-Material in Kontakt mit wenigstens einem organischen Akzeptor-Material aufweist, wobei das Donor-Material und das Akzeptor-Material einen Donor-Akzeptor-Heteroübergang, im speziellen sogenannte bulk heterojunction (BHJ), ausbilden, und wobei die photoaktive Schicht mindestens eine erfindungsgemäße Verbindung aufweist.

In einer bevorzugten Ausführungsform der Erfindung weist das optoelektronische Bauelement mindestens eine weitere Schicht auf, bevorzugt mindestens eine Ladungstransportschicht, insbesondere eine Elektronentransportschicht und/oder eine Lochtransportschicht.

In einer bevorzugten Ausführungsform der Erfindung weist die mindestens eine Ladungstransportschicht, insbesondere mindestens eine Elektronentransportschicht und/oder mindestens eine Lochtransportschicht, die mindestens eine erfindungsgemäße Verbindung auf.

In einer bevorzugten Ausführungsform der Erfindung weist das optoelektronische Bauelement ein Substrat auf, wobei die erste Elektrode oder die zweite Elektrode auf dem Substrat angeordnet ist, insbesondere kann eine der Elektroden des optoelektronischen Bauelements direkt auf dem Substrat aufgebracht sein, und wobei das Schichtsystem zwischen der ersten Elektrode und der zweiten Elektrode angeordnet ist.

In einer bevorzugten Ausführungsform der Erfindung ist die organische Solarzelle eine Einzel-, eine Tandem-, eine Tripel-, eine Quadrupel- oder eine andere Mehrfachzelle.

Unter einer Tandemzelle wird insbesondere verstanden, dass zwei funktionale Zellen räumlich übereinander gestapelt und in Reihe verschaltet sind, bevorzugt zwischen einer ersten Elektrode und einer zweiten Elektrode, wobei zwischen den Zellen eine oder mehrere Zwischenschichten angeordnet sein können. Unter einer Tripel-, einer Quadrupel- oder einer anderen Mehrfachzelle wird entsprechend verstanden, dass mehr als zwei funktionale Zellen räumlich übereinander gestapelt und in Reihe verschaltet sind, wobei zwischen den Zellen eine Zwischenschicht angeordnet sein kann.

Gemäß einer Weiterbildung der Erfindung ist vorgesehen, dass die photoaktive Schicht als Mischschicht aus der mindestens einen erfindungsgemäßen Verbindung und mindestens einer weiteren Verbindung, oder als Mischschicht der mindestens einen erfindungsgemäßen Verbindung und mindestens zwei weiteren Verbindungen ausgebildet ist, wobei die Verbindungen Absorbermaterialien sind.

In einer bevorzugten Ausführungsform der Erfindung ist das optoelektronische Bauelement als eine nip, ni, ip, pnip, pni, pip, nipn, nin, ipn, pnipn, oder pipn-Zelle ausgebildet, oder als eine Kombination aus nip, ni, ip, pnip, pni, pip, nipn, nin, ipn, pnipn, oder pipn-Zellen ausgebildet.

In einer besonders bevorzugten Ausführungsform der Erfindung ist das optoelektronische Bauelement als eine mip-Zelle ausgebildet, oder als eine Kombination einer mip-Zelle mit einer nip, ni, ip, pnip, pni, pip, nipn, nin, ipn, pnipn, oder pipn-Zelle ausgebildet.

Unter einer i-Schicht wird insbesondere eine intrinsische undotierte Schicht verstanden. Eine oder mehrere i-Schichten können dabei aus einem Material (planar heterojunctions, PHJ) oder auch aus einer Mischung zweier oder mehrerer Materialien (bulk heterojunctions, BHJ) bestehen, die ein interpenetrierendes Netzwerk aufweisen.

In einer bevorzugten Ausführungsform ist die erfindungsgemäße Verbindung und/oder eine Schicht mit der mindestens einen erfindungsgemäßen Verbindung mittels Vakuumprozessierung, Gasphasenabscheidung oder Lösungsmittel-Prozessierung abscheidbar, insbesondere bevorzugt mittels Vakuumprozessierung.

Die Aufgabe der vorliegenden Erfindung wird auch gelöst, indem die Verwendung einer erfindungsgemäßen Verbindung in einem optoelektronischen Bauelement, bevorzugt einem organischen optoelektronischen Bauelement, bereitgestellt wird, insbesondere nach einem der zuvor beschriebenen Ausführungsbeispiele. Dabei ergeben sich für die Verwendung der erfindungsgemäßen Verbindung in einem optoelektronischen Bauelement insbesondere die Vorteile, die bereits in Zusammenhang mit der erfindungsgemäßen Verbindung und dem optoelektronischen Bauelement mit der mindestens einen erfindungsgemäßen Verbindung erläutert wurden.

In einer bevorzugten Ausführungsform der Erfindung ist das optoelektronische Bauelement ein organisches optoelektronisches Bauelement, bevorzugt eine organische Solarzelle.

Im Folgenden werden einige Ausführungsbeispiele erfindungsgemäßer Verbindungen und nicht-erfindungsgemäßer Verbindungen (F1 und F2) mit deren optischen Eigenschaften gezeigt. Tabelle 1 zeigt in einer Übersicht, Schmelzpunkte und Absorptionsmaxima (in nm und eV im Lösungsmittel (LM)) dieser Verbindungen. Die spektralen Daten beziehen sich auf 30nm dicke Vakuumaufdampfschichten auf Quarzglas. Die zugehörigen Absorptionsspektren der in Tabelle 1 gelisteten Verbindungen F3 und F12 sind in den Figuren 3 und 4 und ein Vergleich mit der Vergleichsverbindung F1 in Figur 5 gezeigt.

**Tabelle 1**

| **Nr.** | **Struktur** | **Smp. /°C^{a}** | **λmax (LM)/nm^{b}** | **λmax (LM)/eV^{b}** |
|---|---|---|---|---|
| F1 | 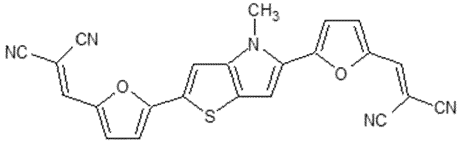 | ca. 370°C | 562 ^{c} | 2,21 ^{c} |
| F2 | 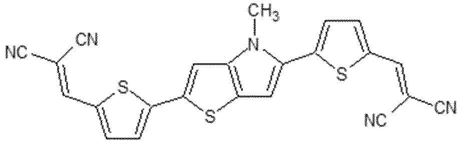 | 349 | 555 ^{b} | 2,23 ^{b} |
| F3 | 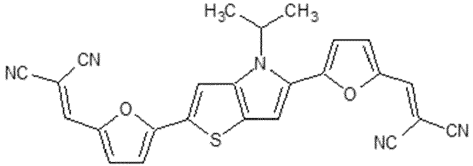 | 356 | 569 ^{b} | 2,18 ^{b} |
| F4 | 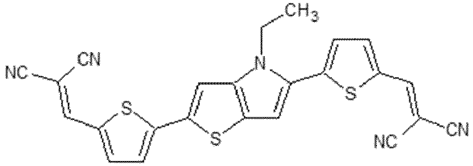 | 330 | 543 ^{b} | 2,28 ^{b} |
| F5 | 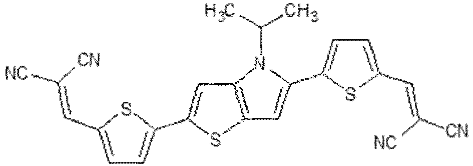 | 343 | 540 ^{c} | 2.30 ^{c} |
| F6 | 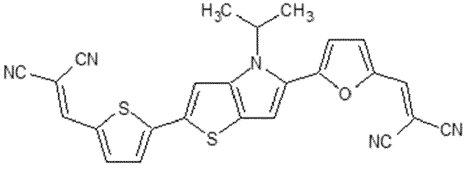 | nd | 558 ^{c} | 2,22 ^{c} |
| F7 | 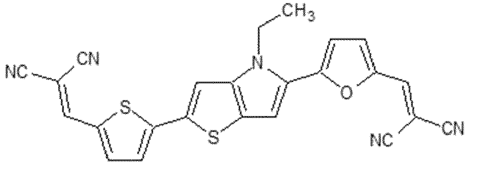 | nd | | |
| F8 | 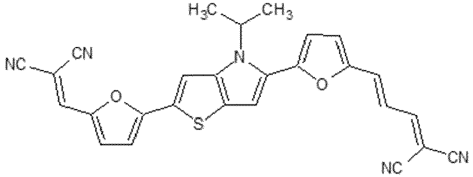 | nd | | |
| F9 | 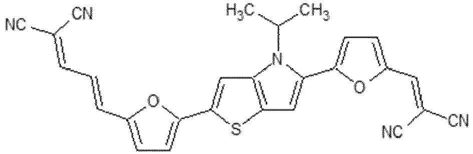 | nd | | |
| F10 | 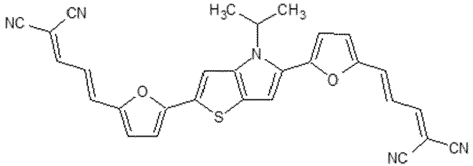 | 325 | 597 ^{b} | 2,08 ^{b} |
| F11 | 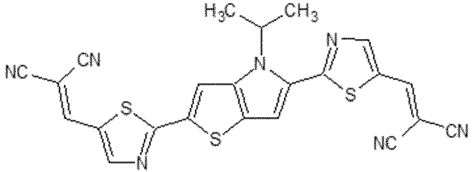 | 314 | 534 ^{b} | 2,32 ^{b} |
| F12 | 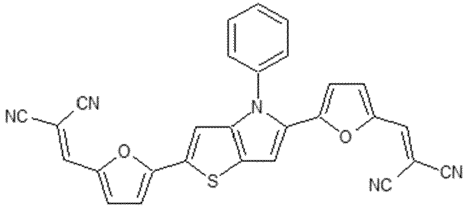 | 291 | 555 ^{b} | 2,23 ^{b} |

| | | | | |
|---|---|---|---|---|
| ^{a} onset DSC (dynamische Differenzkalorimetrie) ^{b} in Dichlormethan | | | | |

Die optischen Eigenschaften wurden experimentell bestimmt. Die Absorptionsmaxima λmax wurde mit einer verdünnten Lösung in einer Küvette in Dichlormethan mittels eines Photometers bestimmt. Die gemessenen Absorptionsmaxima aller beschriebenen Verbindungen liegen in einem Bereich von 534 bis 597 nm.

Tabelle 2 zeigt verschiedene Parameter der erfindungsgemäßen Verbindungen F3, F6 und F12 und der Vergleichsverbindung F1. Die Parameter Leerlaufspannung Uoc, Kurzschlussstrom Jsc und Füllfaktor FF beziehen sich jeweils auf den gleichen Aufbau einer Solarzelle.

Zur Untersuchung der Verbindungen, also deren Einsatz als Absorbermaterialien in organischen optoelektronischen Bauelementen, wurde die Strom-Spannungskurve in einer BHJ-Zelle mit dem Aufbau: Glas mit ITO / C60 (15 nm) / Absorbermaterial:C60 (30nm, 3:2, 50°C) / BPAPF (10nm) / BPAPF:NDP9 (30nm, 10%wt NDP9) / NDP9 (1nm) / Al (100nm) unter AM1.5 Beleuchtung gemessen (AM = Air Mass; AM = 1,5 bei diesem Spektrum beträgt die globale Strahlungsleistung 1000 W/m²; AM = 1,5 als Standardwert für die Vermessung von Solarmodulen), wobei die photoaktive Schicht ein Volumenheteroübergang (bulk heterojunction - BHJ) ist. Auf ein GlasSubstrat ist ein transparenter Deckkontakt aus ITO (Indium-ZinnOxid) aufgebracht. ITO dient dabei als Elektrode, und das benachbarte Fulleren C60 als Elektronentransportschicht (ETL), daran schließt sich die photoaktive Schicht C60 als Elektronenakzeptormaterial und der jeweiligen Verbindung an, gefolgt von BPAPF (9,9-bis[4-(N,N-bis-biphenyl-4-yl-amino)phenyl]-9H-fluoren) als Lochtransportschicht (HTL) und mit NDP9 (Novaled AG) dotiertem BPAPF, gefolgt von einer Elektrode aus Aluminium.

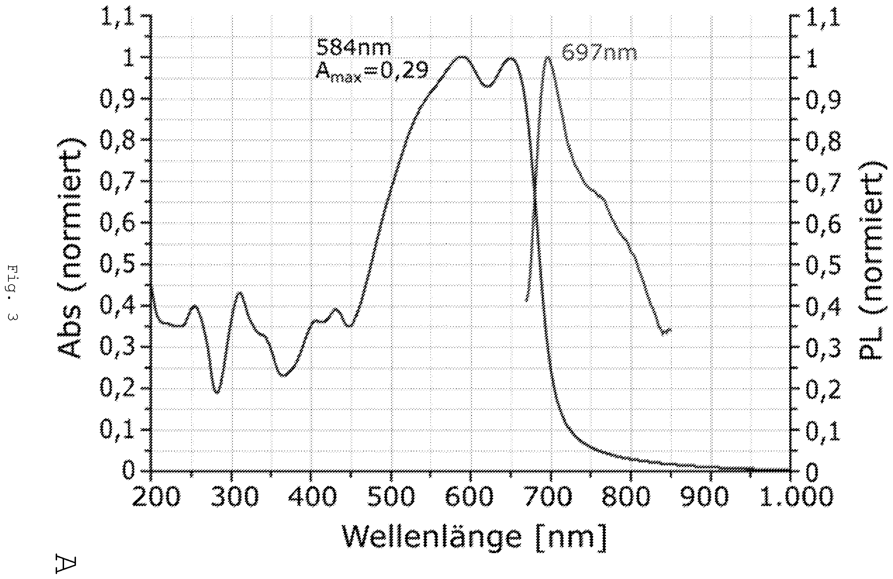

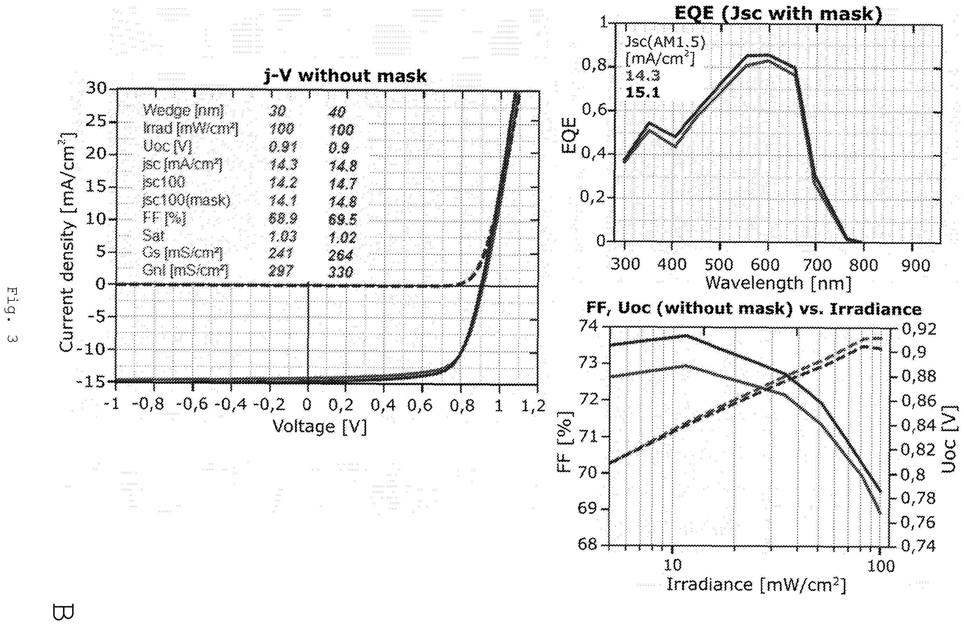

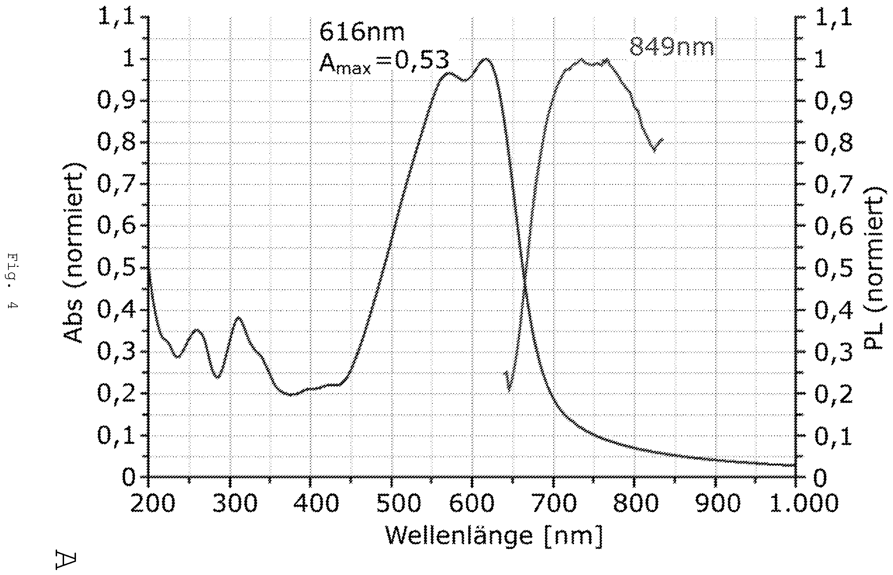

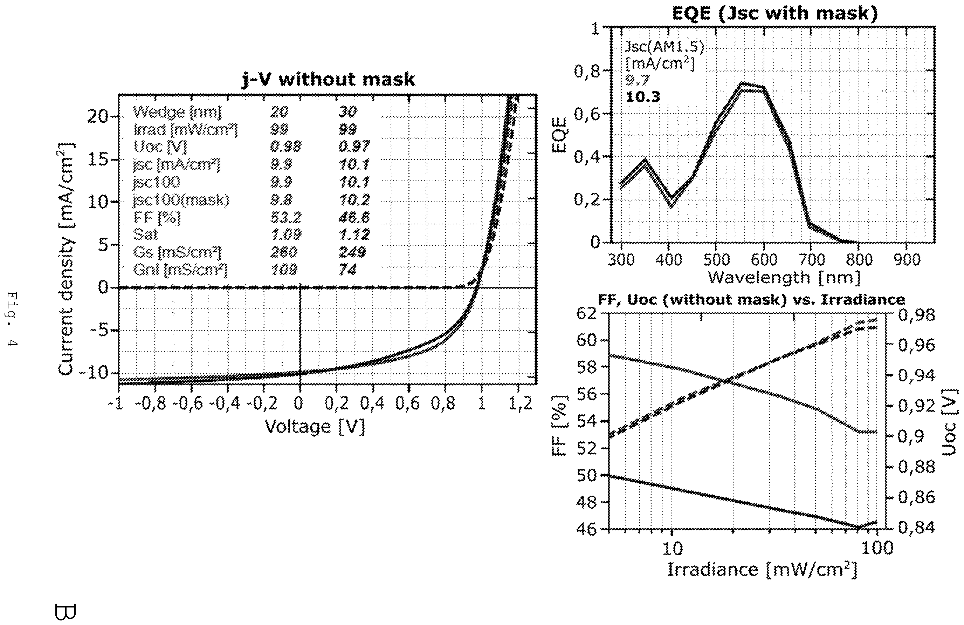

Der vorteilhafte technische Effekt von erfindungsgemäßen Verbindungen, welche eine zentrale Thienopyrrol-Einheit oder eine zentrale Furopyrrol-Einheit, insbesondere eine zentrale Thienopyrrol-Einheit, in Wechselwirkung mit einer Furan-Gruppe oder einer Thiophen-Gruppe, insbesondere einer Furan-Gruppe, an dem Pyrrol der zentralen Thienopyrrol-Einheit oder der zentralen Furopyrrol-Einheit aufweisen, und die am N des Pyrrols der zentralen Thienopyrrol-Einheit oder der zentralen Furopyrrol-Einheit eine sterisch anspruchsvolle Gruppe aufweisen, ist im Folgenden in Tabelle 2 beispielhaft anhand der Verbindung F3, F6 und F12 gegenüber einer Vergleichsverbindung F1 aufgezeigt. Die experimentellen Daten der Verbindungen F3 und F12 sind in Fig. 3 und 4 gezeigt.

**Tabelle 2**

| **Substanz** | **Steilheit** | **Uoc [V]** | **Jsc /EQE [mA/cm²]** | **FF [%]** |
|---|---|---|---|---|
| 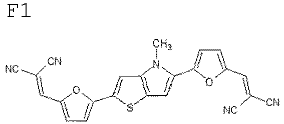 | 2,75 | 0,81 | 7,5 | 47,0 |
| 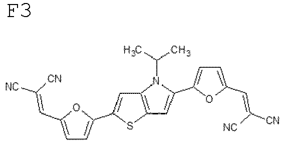 | 9,72 | 0,91 | 15,1 | 68,9 |
| 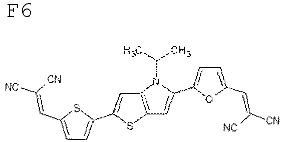 | 5,76 | | | |
| 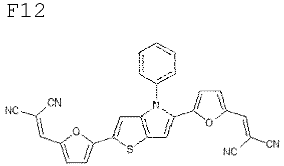 | 5, 9 | 0,97 | 10,3 | 46, 6 |

Die besonders vorteilhaften Eigenschaften der erfindungsgemäßen Verbindungen zeigen sich bei identischem Solarzellenaufbau auch in den Parametern Leerlaufspannung Uoc, Kurzschlussstrom Jsc und Füllfaktor FF. Die erfindungsgemäßen Verbindungen weisen nicht nur verbesserte Absorptionseigenschaften auf, sondern auch geeignete Ladungstransporteigenschaften. In Abhängigkeit der Ladungstransporteigenschaften und der Absorptionseigenschaften können hohe Photoströme mit guten Füllfaktoren erreicht werden. Damit lassen sich besonders gute Kombinationen photoaktiver Schichten herstellen, insbesondere für Tandem-, Tripel-, Quadrupel-, oder Mehrfach-Solarzellen.

Die erfindungsgemäßen Verbindungen weisen dazu in einer besonders bevorzugten Ausführungsform eine zentrale Thienopyrrol-Einheit mit einem Furan-Ring am Pyrrol der zentralen Thienopyrrol-Einheit zusammen mit einer sterisch anspruchsvollen Gruppe am N des Pyrrol-Rings der zentralen Thienopyrrol-Einheit auf, beispielhaft sind die Verbindungen F3 und F6 in Tabelle 2 dargestellt.

Die Erfindung wird im Folgenden anhand der Zeichnungen näher erläutert. Dabei zeigen:

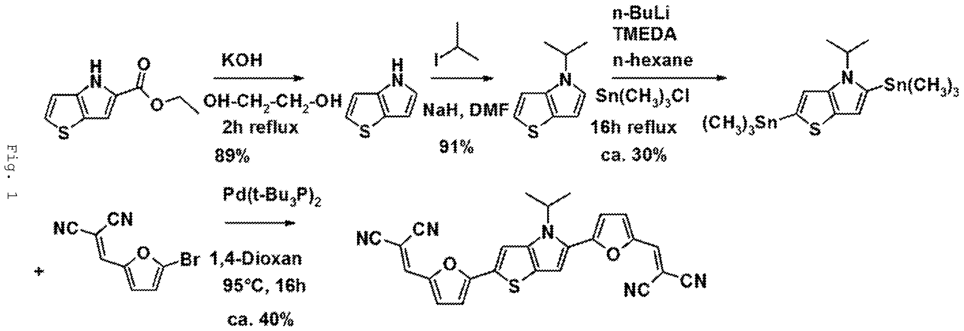

Fig. 1 ein Ausführungsbeispiel eines Syntheseschemas zur Synthese von erfindungsgemäßen Verbindungen;

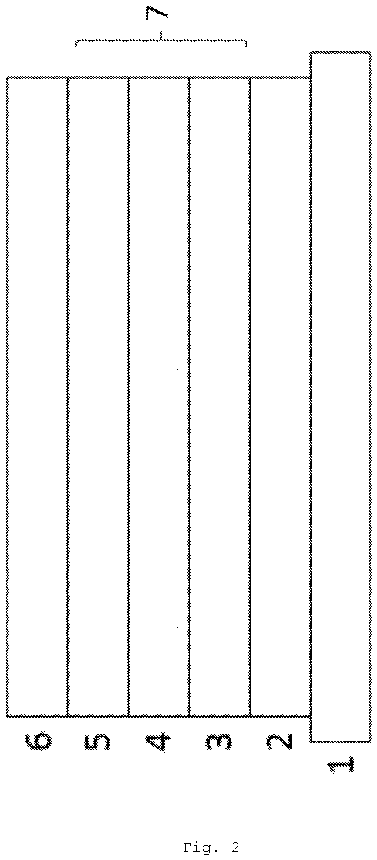

Fig. 2 eine schematische Darstellung eines Ausführungsbeispiels eines optoelektronischen Bauelements im Querschnitt;
Fig. 3 eine grafische Darstellung des Absorptionsspektrums von Verbindung F3, und der Strom-Spannungskurve, der spektralen externen Quantenausbeute und des Füllfaktors einer BHJ-Zelle mit der Verbindung F3, gemessen an einem organischen optoelektronischen Bauelement in Form einer organischen Solarzelle;
Fig. 4 eine grafische Darstellung des Absorptionsspektrums von Verbindung F12, und der Strom-Spannungskurve, der spektralen externen Quantenausbeute und des Füllfaktors einer BHJ-Zelle mit der Verbindung F12, gemessen an einem organischen optoelektronischen Bauelement in Form einer organischen Solarzelle;

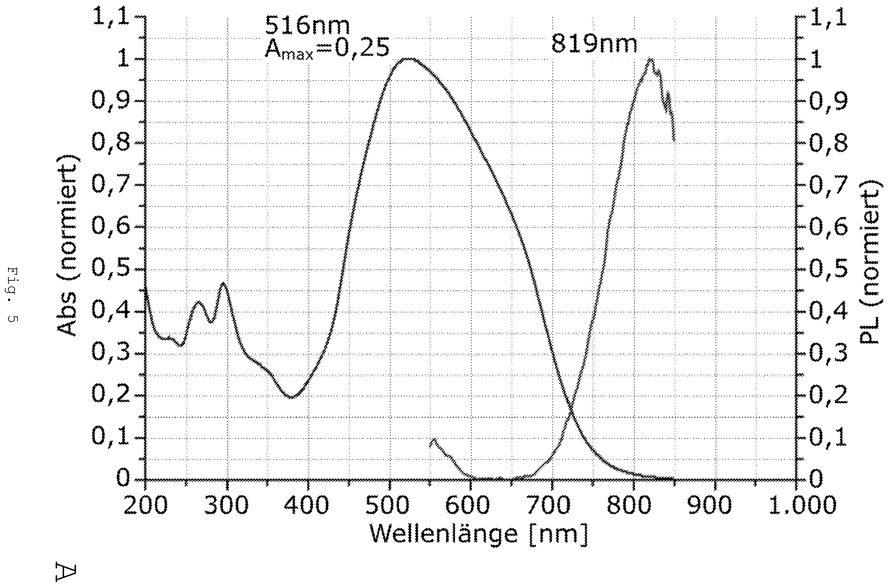

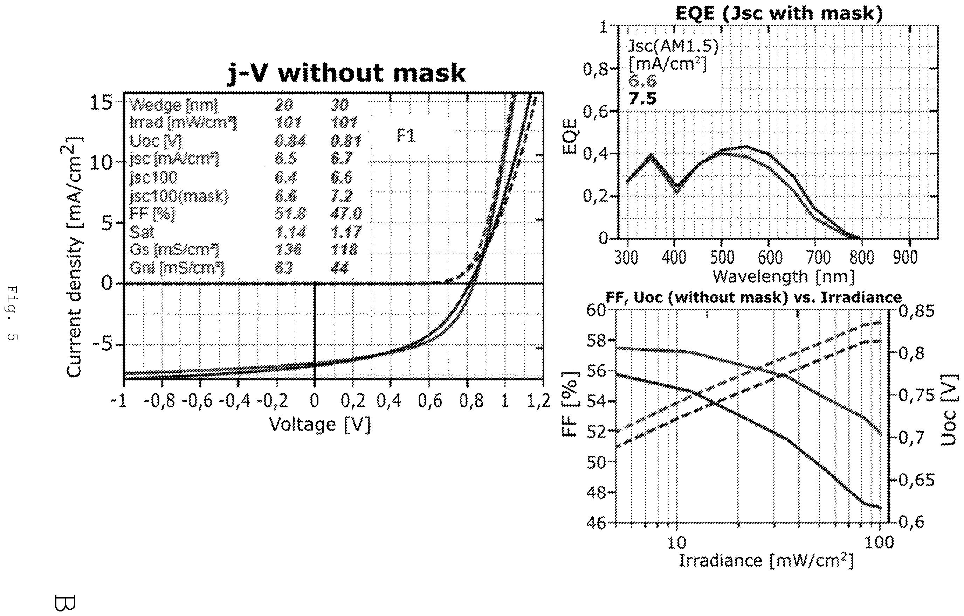

Fig. 5 eine grafische Darstellung des Absorptionsspektrums von Vergleichsverbindung F1, und der Strom-Spannungskurve, der spektralen externen Quantenausbeute und des Füllfaktors einer BHJ-Zelle mit der Vergleichsverbindung F1, gemessen an einem organischen optoelektronischen Bauelement in Form einer organischen Solarzelle; und

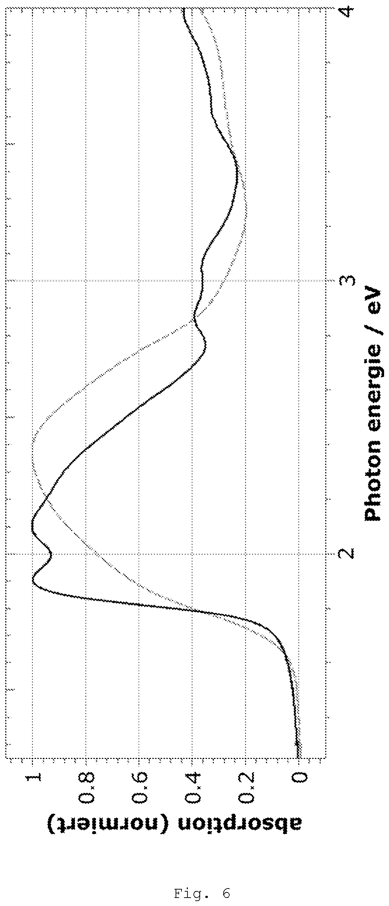

Fig. 6 eine grafische Darstellung eines Vergleichs des Absorptionsspektrums der Verbindung F3 mit einer Vergleichsverbindung F1.

### Ausführungsbeispiele

Fig. 1 zeigt ein Ausführungsbeispiel eines Syntheseschemas zur Synthese von erfindungsgemäßen Verbindungen.

Die Herstellung der erfindungsgemäßen Verbindung ist dem Fachmann aus dem Stand der Technik bekannt. In diesem Zusammenhang wird insbesondere auf die internationalen Patentanmeldungen WO2017114937A1 und WO2017114938A1 verwiesen. Die erfindungsgemäßen Verbindungen sind dadurch einfach und in guten Ausbeuten zugänglich. Beispielhaft ist im Folgenden eine allgemeine Synthese zur Herstellung einer erfindungsgemäßen Verbindung dargestellt.

Die erfindungsgemäße Verbindung der allgemeinen Formel I 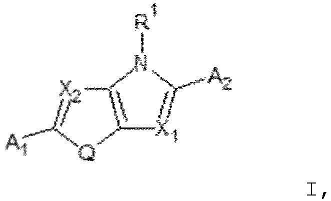 ist dadurch gekennzeichnet, dass
Q O oder S ist, bevorzugt ist Q S;
R1 ausgewählt ist aus der Gruppe bestehend aus Alkoxy, Alkyl, fluoriertem Alkyl, teilfluoriertem Alkyl, und Aryl;
X1 und X2 unabhängig voneinander N oder C-R4 sind; wobei
R4 ausgewählt ist aus der Gruppe bestehend aus H, Halogen, Alkoxy, verzweigtem oder linearem, zyklischem oder offenkettigem Alkyl, Alkenyl, und Aryl, wobei H jeweils substituiert sein kann;
A1 eine Gruppe mit der Formel Ia 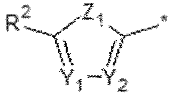 ist,
wobei * die Anknüpfung an die Verbindung der allgemeinen Formel I bezeichnet; Z1 ausgewählt ist aus der Gruppe bestehend aus O, S, Se, und N-R5, wobei R5 ausgewählt ist aus der Gruppe bestehend aus H, Alkoxy, Alkyl, fluoriertem Alkyl, teilfluoriertem Alkyl, und Aryl;
Y1 N oder C-R6 ist, wobei R6 ausgewählt ist aus der Gruppe bestehend aus H, Halogen, Alkoxy, verzweigtem oder linearem, zyklischem oder offenkettigem Alkyl, Alkenyl, und Aryl, wobei H jeweils substituiert sein kann; Y2 N oder C-R7 ist, wobei R7 ausgewählt ist aus der Gruppe bestehend aus H, Halogen, Alkoxy, verzweigtem oder linearem, zyklischem oder offenkettigem Alkyl, Alkenyl, und Aryl, wobei H jeweils substituiert sein kann; und wobei R6 und R7 miteinander in Form einer Ringstruktur homocyclisch oder heterocyclisch verbunden sein können; R2 ausgewählt ist aus der Gruppe bestehend aus H, Halogen, Alkoxy, Alkyl, fluoriertem Alkyl, teilfluoriertem Alkyl, verzweigtem oder linearem, zyklischem oder offenkettigem Alkyl, Amino, Aryl, Heteroaryl, Alkenyl, und einer elektronenziehenden Alkyl-Gruppe mit zumindest einer C=C-Doppelbindung, wobei H durch CN oder F substituiert sein kann; A2 eine Gruppe mit der Formel Ib 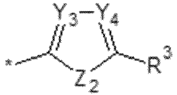 ist,
wobei * die Anknüpfung an die Verbindung der allgemeinen Formel I bezeichnet; Z2 ausgewählt ist aus der Gruppe bestehend aus O, S, Se, und N-R8, wobei R8 ausgewählt ist aus der Gruppe bestehend aus H, Alkoxy, Alkyl, fluoriertem Alkyl, teilfluoriertem Alkyl, und Aryl; Y3 N oder C-R9 ist, wobei R9 ausgewählt ist aus der Gruppe bestehend aus H, Halogen, Alkoxy, verzweigtem oder linearem, zyklischem oder offenkettigem Alkyl, Alkenyl, und Aryl, wobei H jeweils substituiert sein kann; Y4 N oder C-R10 ist, wobei R10 ausgewählt ist aus der Gruppe bestehend aus H, Halogen, Alkoxy, verzweigtem oder linearem, zyklischem oder offenkettigem Alkyl, Alkenyl, und Aryl, wobei H jeweils substituiert sein kann; und wobei R9 und R10 miteinander in Form einer Ringstruktur homocyclisch oder heterocyclisch verbunden sein können; und R3 ausgewählt ist aus der Gruppe bestehend aus H, Halogen, Alkoxy, Alkyl, fluoriertem Alkyl, teilfluoriertem Alkyl, verzweigtem oder linearem, zyklischem oder offenkettigem Alkyl, Amino, Aryl, Heteroaryl, Alkenyl, und einer elektronenziehenden Alkyl-Gruppe mit zumindest einer C=C-Doppelbindung, wobei H durch CN oder F substituiert sein kann.

Durch verbesserte Absorptionseigenschaften, insbesondere mit einer steilen Absorption im bathochromen Bereich, in Verbindung mit sehr guten Ladungstransporteigenschaften können hohe Photoströme erzeugt werden. Damit können insbesondere Tandem-, Tripel-, Quadrupel- oder Mehrfachzellen hergestellt werden.

In einer Ausgestaltung der Erfindung ist R1 ausgewählt aus der Gruppe bestehend aus Ethyl, Propyl, Butyl, verzweigtem Alkyl, und Aryl, bevorzugt aus iso-Propyl, iso-Butyl, sec-Butyl, tert-Butyl, iso-Pentyl und Phenyl; und wobei Z1 und Z2 jeweils unabhängig voneinander O oder S sind, bevorzugt ist Z2 O. Dadurch verwirklichen sich die vorteilhaften Effekte der vorliegenden Erfindung in besonderer Weise.

In einer weiteren Ausgestaltung der Erfindung ist die Verbindung eine Verbindung der allgemeinen Formel II, 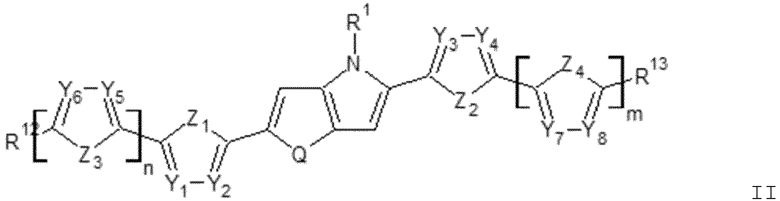
wobei n 0, 1 oder 2 ist und m 0, 1 oder 2 ist;
Q O oder S ist, bevorzugt ist Q S; Z3 und Z4 jeweils unabhängig voneinander ausgewählt sind aus der Gruppe bestehend aus O, S, Se, und N-R11, wobei R11 ausgewählt ist aus der Gruppe bestehend aus H, Alkoxy, Alkyl, fluoriertem Alkyl, teilfluoriertem Alkyl, und Aryl;
R12 ausgewählt ist aus der Gruppe bestehend aus H, Alkoxy, Alkyl, fluoriertem Alkyl, teilfluoriertem Alkyl, Alkenyl, und einer elektronenziehenden Alkyl-Gruppe mit zumindest einer C=C-Doppelbindung, wobei H durch CN oder F substituiert sein kann;
R13 ausgewählt ist aus der Gruppe bestehend aus H, Alkoxy, Alkyl, fluoriertem Alkyl, teilfluoriertem Alkyl, Alkenyl, und einer elektronenziehenden Alkyl-Gruppe mit zumindest einer C=C-Doppelbindung, wobei H durch CN oder F substituiert sein kann; Y5 N oder C-R14 ist, wobei R14 ausgewählt ist aus der Gruppe bestehend aus H, Halogen, Alkoxy, Alkyl, und Alkenyl, wobei H jeweils substituiert sein kann, wobei Y5 bevorzugt N, CH oder CF ist; Y6 N oder C-R15 ist, wobei R15 ausgewählt ist aus der Gruppe bestehend aus H, Halogen, Alkoxy, Alkyl, und Alkenyl, wobei H jeweils substituiert sein kann, wobei Y6 bevorzugt N, CH oder CF ist; Y7 N oder C-R16 ist, wobei R16 ausgewählt ist aus der Gruppe bestehend aus H, Halogen, Alkoxy, Alkyl, und Alkenyl, wobei H jeweils substituiert sein kann, wobei Y7 bevorzugt N, CH oder CF ist; Y8 N oder C-R17 ist, wobei R17 ausgewählt ist aus der Gruppe bestehend aus H, Halogen, Alkoxy, Alkyl, und Alkenyl, wobei H jeweils substituiert sein kann, wobei Y8 bevorzugt N, CH oder CF ist; und wobei jeweils zusammen R14 und R15, und/oder R16 und R17 einen homocyclischen Fünf-Ring oder Sechs-Ring, oder einen heterocyclischen Fünf-Ring oder Sechs-Ring mit mindestens einem Heteroatom ausgewählt aus der Gruppe bestehend aus S, O und N bilden können.

In einer weiteren Ausgestaltung der Erfindung bilden R14 und R15 zusammen und/oder R16 und R17 zusammen einen homocyclischen Fünf-Ring oder Sechs-Ring oder einen heterocyclischen Fünf-Ring oder Sechs-Ring mit mindestens einem Heteroatom ausgewählt aus der Gruppe bestehend aus S, O und N.

In einer weiteren Ausgestaltung der Erfindung sind Z3 und Z4 unabhängig voneinander O oder S.

In einer weiteren Ausgestaltung der Erfindung ist A1 eine Gruppe mit der Formel IIa 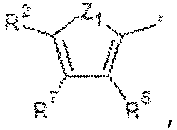
wobei R6 ausgewählt ist aus der Gruppe bestehend aus H, Halogen, Alkoxy, verzweigtem oder linearem, zyklischem oder offenkettigem Alkyl, Alkenyl, und Aryl, wobei H jeweils substituiert sein kann;
R7 ausgewählt ist aus der Gruppe bestehend aus H, Halogen, Alkoxy, verzweigtem oder linearem, zyklischem oder offenkettigem Alkyl, Alkenyl, und Aryl, wobei H jeweils substituiert sein kann; und wobei
R6 und R7 zusammen einen homocyclischen Fünf-Ring oder Sechs-Ring, oder einen heterocyclischen Fünf-Ring oder Sechs-Ring mit mindestens einem Heteroatom ausgewählt aus der Gruppe bestehend aus S, O und N bilden können;
und A2 eine Gruppe mit der Formel IIb 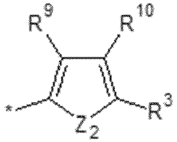 ist,
wobei R9 ausgewählt ist aus der Gruppe bestehend aus H, Halogen, Alkoxy, verzweigtem oder linearem, zyklischem oder offenkettigem Alkyl, Alkenyl, und Aryl, wobei H jeweils substituiert sein kann; R10 ausgewählt ist aus der Gruppe bestehend aus H, Halogen, Alkoxy, verzweigtem oder linearem, zyklischem oder offenkettigem Alkyl, Alkenyl, und Aryl, wobei H jeweils substituiert sein kann; und wobei R9 und R10 zusammen einen homocyclischen Fünf-Ring oder Sechs-Ring, oder einen heterocyclischen Fünf-Ring oder Sechs-Ring mit mindestens einem Heteroatom ausgewählt aus der Gruppe bestehend aus S, O und N bilden können.

In einer weiteren Ausgestaltung der Erfindung ist die Verbindung eine Verbindung der allgemeinen Formel III, IV und/oder V, 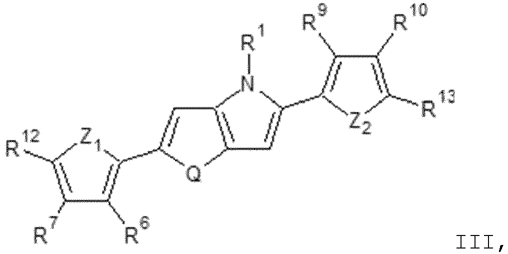 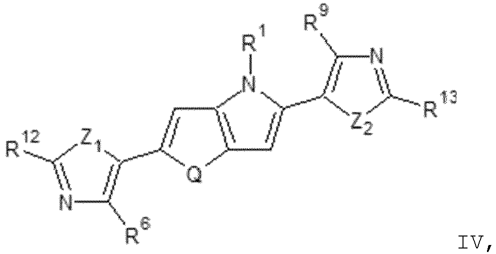 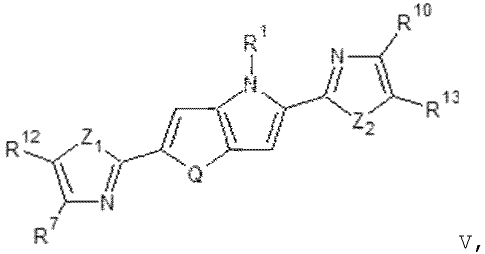
wobei Q O oder S ist, bevorzugt ist Q S; Z1 und Z2 jeweils unabhängig voneinander O oder S sind, bevorzugt ist Z2 O; R6 ausgewählt ist aus der Gruppe bestehend aus H, Halogen, Alkoxy, Alkyl, und Alkenyl, wobei H jeweils substituiert sein kann;
R7 ausgewählt ist aus der Gruppe bestehend aus H, Halogen, Alkoxy, Alkyl, und Alkenyl, wobei H jeweils substituiert sein kann; und
wobei R6 und R7 zusammen einen homocyclischen Fünf-Ring oder Sechs-Ring, oder einen heterocyclischen Fünf-Ring oder Sechs-Ring mit mindestens einem Heteroatom ausgewählt aus der Gruppe bestehend aus S, O und N bilden können; R9 ausgewählt ist aus der Gruppe bestehend aus H, Halogen, Alkoxy, Alkyl, und Alkenyl, wobei H jeweils substituiert sein kann; R10 ausgewählt ist aus der Gruppe bestehend aus H, Halogen, Alkoxy, Alkyl, und Alkenyl, wobei H jeweils substituiert sein kann; und wobei R9 und R10 zusammen einen homocyclischen Fünf-Ring oder Sechs-Ring, oder einen heterocyclischen Fünf-Ring oder Sechs-Ring mit mindestens einem Heteroatom ausgewählt aus der Gruppe bestehend aus S, O und N bilden können; R12 ausgewählt ist aus der Gruppe bestehend aus H, Alkoxy, Alkyl, fluoriertem Alkyl, teilfluoriertem Alkyl, Alkenyl, und einer elektronenziehenden Alkyl-Gruppe mit zumindest einer C=C-Doppelbindung, wobei H durch CN oder F substituiert sein kann; und
R13 ausgewählt ist aus der Gruppe bestehend aus H, Alkoxy, Alkyl, fluoriertem Alkyl, teilfluoriertem Alkyl, Alkenyl, und einer elektronenziehenden Alkyl-Gruppe mit zumindest einer C=C-Doppelbindung, wobei H durch CN oder F substituiert sein kann.

In einer weiteren Ausgestaltung der Erfindung sind R2 und R3, oder R12 und R13 jeweils unabhängig voneinander 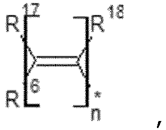 wobei n 1, 2 oder 3 ist, wobei * die Anknüpfung der Gruppe R2, R3, R12 und/oder R13 bezeichnet; R16, R17 und R18 jeweils unabhängig voneinander ausgewählt sind aus der Gruppe bestehend aus H, Halogen, CN, COO-Alkyl, Alkyl und Alkenyl, mit der Bedingung, dass R16 und R17 nicht beide H sind, wobei bevorzugt R16 und R17 CN sind.

In einer weiteren Ausgestaltung der Erfindung sind R2 und R3, oder R12 und R13 jeweils unabhängig voneinander ausgewählt aus der Gruppe bestehend aus: 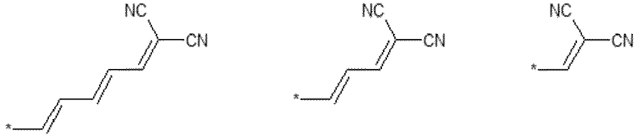 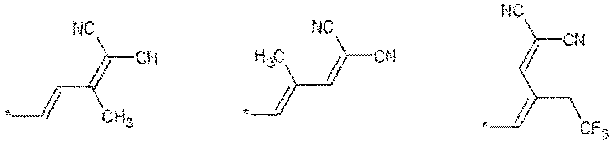 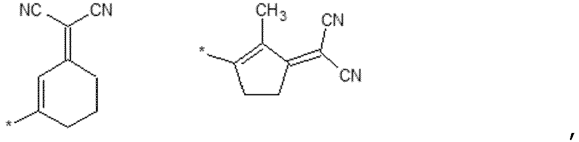 wobei * die Anknüpfung der Gruppe R2, R3, R12 und/oder R13 bezeichnet, wobei CN durch F ersetzt sein kann, und wobei bevorzugt R2 und R3, oder R12 und R13 gleich sind.

In einer weiteren Ausgestaltung der Erfindung sind R6 und R7 H, R9 und R10 H, und Z2 O, wobei bevorzugt Z1 und Z2 O sind.

In einer weiteren Ausgestaltung der Erfindung ist die Verbindung ausgewählt aus der Gruppe bestehend aus:

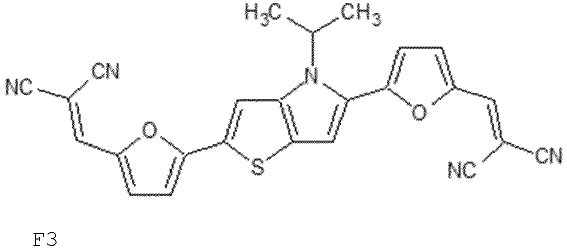

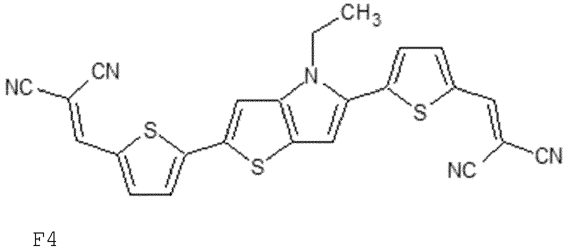

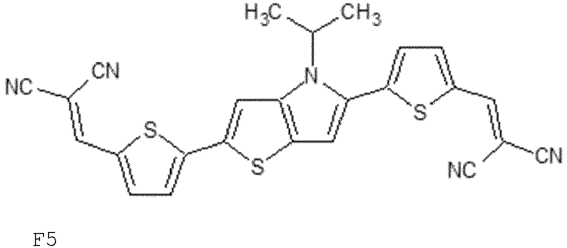

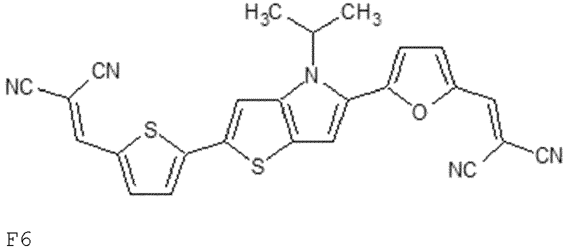

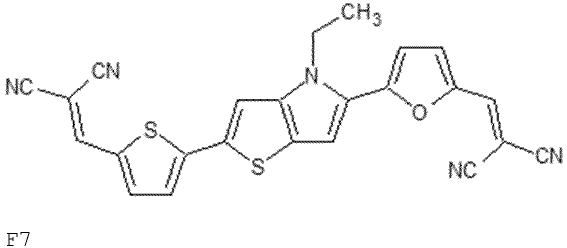

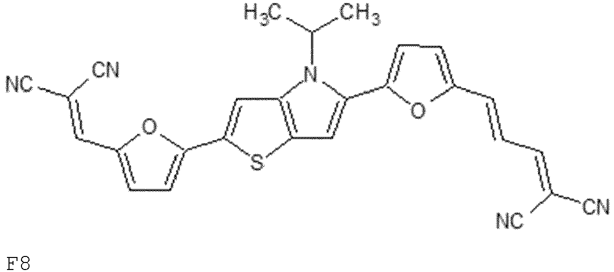

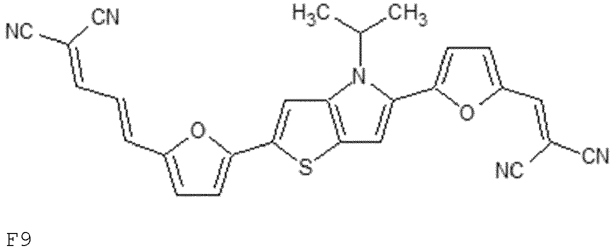

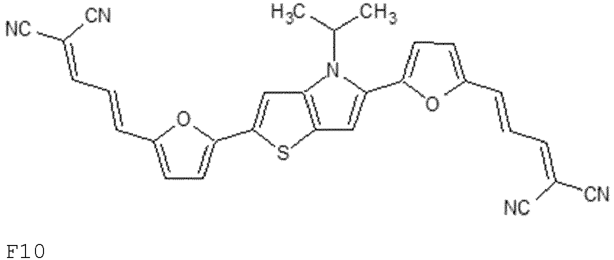

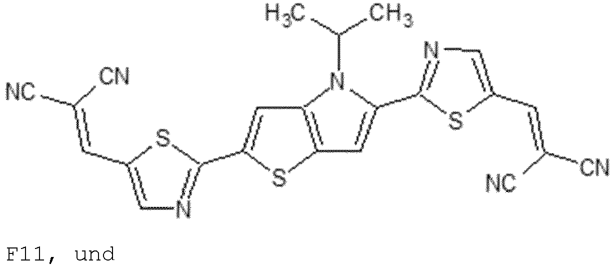

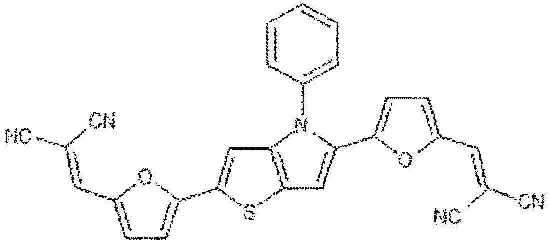

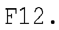

In einer weiteren Ausgestaltung der Erfindung ist die Gruppe A1 gleich der Gruppe A2.

Fig. 2 zeigt eine schematische Darstellung eines Ausführungsbeispiels eines optoelektronischen Bauelements im Querschnitt.

Das Optoelektronische Bauelement umfasst eine erste Elektrode 2, eine zweite Elektrode 6 und ein Schichtsystem 7, wobei das Schichtsystem 7 zwischen der ersten Elektrode 2 und der zweiten Elektrode 6 angeordnet ist. Dabei weist mindestens eine Schicht des Schichtsystems 7 mindestens eine erfindungsgemäße Verbindung auf.

In einer Ausgestaltung der Erfindung ist das optoelektronische Bauelement ein organisches optoelektronisches Bauelement, bevorzugt eine organische Solarzelle, ein OFET, eine OLED oder ein organischer Photodetektor.

In diesem Ausführungsbeispiel ist das optoelektronische Bauelement eine organische Solarzelle. Die organische Solarzelle weist ein Substrat 1 auf, z. B. aus Glas, auf dem sich eine Elektrode 2 befindet, die z. B. ITO umfasst. Darauf angeordnet ist ein Schichtsystem 7 mit einer elektronentransportierenden Schicht 3 (ETL) sowie einer photoaktiven Schicht 4 mit mindestens einer erfindungsgemäßen Verbindung, einem p-leitenden Donor-Material, und einem n-leitenden Akzeptor-Material, z. B. C60 Fulleren, entweder als flacher Heteroübergang oder als Volumenheteroübergang. Darüber angeordnet befindet sich eine p-dotierte Lochtransportschicht 5 (HTL), und eine Elektrode 6 aus Aluminium.

In einer weiteren Ausgestaltung der Erfindung weist das Schichtsystem 7 mindestens eine photoaktive Schicht 4 auf, bevorzugt eine Absorberschicht, wobei die mindestens eine photoaktive Schicht 4 die mindestens eine erfindungsgemäße Verbindung aufweist.

In einer weiteren Ausgestaltung der Erfindung weist das Schichtsystem 7 mindestens zwei photoaktive Schichten auf, bevorzugt mindestens drei photoaktive Schichten, oder bevorzugt mindestens vier photoaktive Schichten.

In einer weiteren Ausgestaltung der Erfindung ist die photoaktive Schicht 4 als Mischschicht aus der mindestens einen erfindungsgemäßen Verbindung und mindestens einer weiteren Verbindung, oder als Mischschicht der mindestens einen erfindungsgemäßen Verbindung und mindestens zwei weiteren Verbindungen ausgebildet, wobei die Verbindungen Absorbermaterialien sind.

In einer weiteren Ausgestaltung der Erfindung ist das optoelektronische Bauelement als Tandemzelle, Tripelzelle oder Mehrfachzelle ausgebildet.

In den folgenden Figuren 3 und 4 werden konkrete Ausführungsbeispiele der erfindungsgemäßen Verbindungen sowie deren optische Eigenschaften aufgezeigt.

Fig. 3 zeigt eine grafische Darstellung des Absorptionsspektrums von Verbindung F3 (Fig. 3A), und der Strom-Spannungskurve, der spektralen externen Quantenausbeute und des Füllfaktors einer BHJ-Zelle mit der Verbindung F3 (Fig. 3B), gemessen an einem organischen optoelektronischen Bauelement in Form einer organischen Solarzelle.

Die Absorptionsspektren (optische Dichte über Wellenlänge in nm) wurden für 30 nm dicke vakuumaufgedampfte Schichten der Verbindungen F3, F12 und der Vergleichsverbindung F1 jeweils auf Quarzglas gemessen. Die Strom-Spannungskurve enthält Kennzahlen, welche die organische Solarzelle kennzeichnen. Die wichtigsten Kennzahlen sind hierbei der Füllfaktor FF, die Leerlaufspannung Uoc und der Kurzschlussstrom Jsc.

Das Absorptionsspektrum der Verbindung F3 ist in Fig. 3A dargestellt. Die Verbindung F3 zeigt einen besonders steilen Verlauf der Absorption im bathochromen Bereich, also an der Flanke im höheren Wellenlängenbereich des Absorptionsspektrums, mit einer Steilheit von 9,72. Die Steilheit des Verlaufs des Absorptionsspektrums ist dabei über die Steigung der Tangente an dem Wendepunkt bestimmt, der zwischen dem Beginn der Absorption und der ersten Kante der Absorption liegt. Unter dem bathochromen Bereich wird insbesondere ein in den roten Wellenlängenbereich des sichtbaren Lichts, also in den längerwelligen, energieärmeren Bereich des elektromagnetischen Spektrums, verschobener Bereich verstanden. Dieser steile Verlauf im bathochromen Bereich des Absorptionsspektrums ist besonders vorteilhaft bei der Bildung von Tandem-, Triple- oder Mehrfachzellen.

In diesem Ausführungsbeispiel weist die BHJ-Zelle auf der ITO-Schicht eine Schicht von C60 mit einer Schichtdicke von 15 nm auf. Auf diese Schicht wurde die Verbindung F3 zusammen mit C60 in einer Dicke von 30 nm aufgetragen. Dieser Schicht folgt eine Schicht aus BPAPF (9,9-bis[4-(N,N-bis-biphenyl-4-yl-amino)phenyl]-9H-fluoren) in einer Schichtdicke von 10 nm, darauf befindet sich eine weitere Schicht umfassend BPAPF und NDP9 in einer Schichtdicke von 30 nm. Der Anteil an BPAPF in dieser Schicht beträgt 10 Gewichtsprozent, bezogen auf die gesamte Schicht. An diese Schicht schließt sich eine weitere Schicht mit NDP9 in einer Dicke von 1 nm an, woraufhin eine Aluminium-Schicht in einer Dicke von 100 nm folgt. Die Strom-Spannungskurve einer BHJ-Zelle mit dem Aufbau: ITO / C60 (15 nm) / F3:C60 (30nm, 3:2, 50°C) / BPAPF (10nm) / BPAPF:NDP9 (30nm, 10%wt NDP9) / NDP9 (1nm) / Al (100nm) wurde bestimmt, wobei die photoaktive Schicht ein Volumenheteroübergang (bulk heterojunction - BHJ) ist. In dem optoelektronischen Bauelement mit Verbindung F3 beträgt der Füllfaktor FF 68,9 %, die Leerlaufspannung Uoc 0,91 V und der Kurzschlussstrom Jsc 15,1 mA/cm2 (Fig. 3B). Der Zellwirkungsgrad eines solchen optoelektronischen Bauelements, insbesondere einer Solarzelle, mit der Verbindung F3 beträgt 9,4 %.

Die Verbindung F3 zeigt zu dem eine gute Verdampfbarkeit im Vakuum.

Fig. 4 zeigt eine grafische Darstellung des Absorptionsspektrums von Verbindung F12 (Fig. 4A), und der Strom-Spannungskurve, der spektralen externen Quantenausbeute und des Füllfaktors einer BHJ-Zelle mit der Verbindung F12 (Fig. 4B), gemessen an einem organischen optoelektronischen Bauelement in Form einer organischen Solarzelle.

Das Absorptionsspektrum der Verbindung F12 ist in Fig. 4A dargestellt. Die Verbindung F12 zeigt, wie schon zuvor die Verbindung F3, einen besonders steilen Verlauf der Absorption mit einer Steilheit von 5, 9.

Die Strom-Spannungskurve einer BHJ-Zelle mit dem Aufbau: ITO / C60 (15 nm) / F12:C60 (30nm, 3:2, 50°C) / BPAPF (10nm) / BPAPF:NDP9 (30nm, 10%wt NDP9) / NDP9 (1nm) / Al (100nm) wurde bestimmt, wobei die photoaktive Schicht ein Volumenheteroübergang (bulk heterojunction - BHJ) ist. In dem optoelektronischen Bauelement mit Verbindung F12 beträgt der Füllfaktor FF 46,6 %, die Leerlaufspannung Uoc 0,97 V und der Kurzschlussstrom Jsc 10,3 mA/cm2 (Fig. 4B). Der Zellwirkungsgrad eines solchen optoelektronischen Bauelements, insbesondere einer Solarzelle, mit der Verbindung F12 beträgt 4,7 %.

Die Verbindung F12 zeigt zu dem eine gute Verdampfbarkeit im Vakuum.

Die experimentellen Daten der Verbindungen F3 und F12 mit den Absorptionseigenschaften und den in organischen Solarzellen gemessenen Strom-Spannungsverläufen belegen, dass die Verbindungen F3 und F12 für die Anwendung in organischen Solarzellen sowie anderen organischen optoelektronischen Bauelementen sehr gut geeignet sind.

Fig. 5 zeigt eine grafische Darstellung des Absorptionsspektrums von Vergleichsverbindung F1 (Fig. 5A), und der Strom-Spannungskurve, der spektralen externen Quantenausbeute und des Füllfaktors einer BHJ-Zelle mit der Vergleichsverbindung F1 (Fig. 5B), gemessen an einem organischen optoelektronischen Bauelement in Form einer organischen Solarzelle.

Die Verbindung F1 zeigt einen flachen Verlauf der Absorption mit einer Steilheit von 2,75.

Die Strom-Spannungskurve einer BHJ-Zelle mit dem Aufbau: ITO / C60 (15 nm) / F1:C60 (30nm, 3:2, 50°C) / BPAPF (10nm) / BPAPF:NDP9 (30nm, 10%wt NDP9) / NDP9 (1nm) / Al (100nm) wurde bestimmt, wobei die photoaktive Schicht ein Volumenheteroübergang (bulk heterojunction - BHJ) ist. In dem optoelektronischen Bauelement mit Verbindung F1 beträgt der Füllfaktor FF 47,0 %, die Leerlaufspannung Uoc 0,81 V und der Kurzschlussstrom Jsc 7,5 mA/cm2.

Die experimentellen Daten der Vergleichsverbindung F1 und der Verbindungen F3 und F12 zeigen, dass die Verbindungen F3 und F12 bessere Absorptionseigenschaften im Vergleich zu der Vergleichsverbindung F1 aufweisen.

Fig. 6 zeigt eine grafische Darstellung eines Vergleichs des Absorptionsspektrums der Verbindung F3 mit einer Vergleichsverbindung F1.

Die Absorption der Verbindung F3 (dargestellt mit einer durchgezogenen Linie) und der Vergleichsverbindung F1 (dargestellt mit einer gestrichelten Linie) ist gegen die Photonenenergie aufgetragen. Es ist deutlich zu erkennen, dass die Absorption der Verbindung F3 mit einer Steilheit von 9,72 gegenüber der Vergleichsverbindung F1 mit einer Steilheit von 2,75 wesentlich steiler verläuft.

## Patentansprüche

1. Verbindung der allgemeinen Formel I 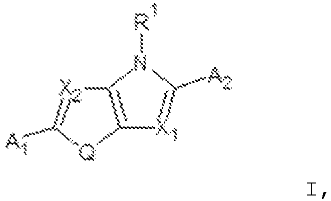 **dadurch gekennzeichnet, dass**
Q O oder S ist, bevorzugt ist Q S;
R1 ausgewählt ist aus der Gruppe bestehend aus Alkoxy, Alkyl, fluoriertem Alkyl, teilfluoriertem Alkyl, und Aryl;
X1 und X2 unabhängig voneinander N oder C-R4 sind; wobei
R4 ausgewählt ist aus der Gruppe bestehend aus H, Halogen, Alkoxy, verzweigtem oder linearem, zyklischem oder offenkettigem Alkyl, Alkenyl, und Aryl, wobei H jeweils substituiert sein kann;
A1 eine Gruppe mit der Formel Ia 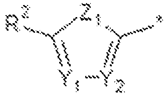 ist,
wobei * die Anknüpfung an die Verbindung der allgemeinen Formel I bezeichnet;
Z1 ausgewählt ist aus der Gruppe bestehend aus O, S, Se, und N-R5, wobei R5 ausgewählt ist aus der Gruppe bestehend aus H, Alkoxy, Alkyl, fluoriertem Alkyl, teilfluoriertem Alkyl, und Aryl;
Y1 N oder C-R6 ist, wobei R6 ausgewählt ist aus der Gruppe bestehend aus H, Halogen, Alkoxy, verzweigtem oder linearem, zyklischem oder offenkettigem Alkyl, Alkenyl, und Aryl, wobei H jeweils substituiert sein kann;
Y2 N oder C-R7 ist, wobei R7 ausgewählt ist aus der Gruppe bestehend aus H, Halogen, Alkoxy, verzweigtem oder linearem, zyklischem oder offenkettigem Alkyl, Alkenyl, und Aryl, wobei H jeweils substituiert sein kann; und wobei R6 und R7 miteinander in Form einer Ringstruktur homocyclisch oder heterocyclisch verbunden sein können;
R2 ausgewählt ist aus der Gruppe bestehend aus H, Halogen, Alkoxy, Alkyl, fluoriertem Alkyl, teilfluoriertem Alkyl, verzweigtem oder linearem, zyklischem oder offenkettigem Alkyl, Amino, Aryl, Heteroaryl, Alkenyl, und einer elektronenziehenden Alkyl-Gruppe mit zumindest einer C=C-Doppelbindung, wobei H durch CN oder F substituiert sein kann;
A2 eine Gruppe mit der Formel Ib 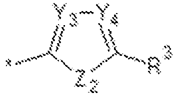 ist,
wobei * die Anknüpfung an die Verbindung der allgemeinen Formel I bezeichnet;
Z2 ausgewählt ist aus der Gruppe bestehend aus O, S, Se, und N-R8, wobei R8 ausgewählt ist aus der Gruppe bestehend aus H, Alkoxy, Alkyl, fluoriertem Alkyl, teilfluoriertem Alkyl, und Aryl;
Y3 N oder C-R9 ist, wobei R9 ausgewählt ist aus der Gruppe bestehend aus H, Halogen, Alkoxy, verzweigtem oder linearem, zyklischem oder offenkettigem Alkyl, Alkenyl, und Aryl, wobei H jeweils substituiert sein kann;
Y4 N oder C-R10 ist, wobei R10 ausgewählt ist aus der Gruppe bestehend aus H, Halogen, Alkoxy, verzweigtem oder linearem, zyklischem oder offenkettigem Alkyl, Alkenyl, und Aryl, wobei H jeweils substituiert sein kann; und wobei R9 und R10 miteinander in Form einer Ringstruktur homocyclisch oder heterocyclisch verbunden sein können; und
R3 ausgewählt ist aus der Gruppe bestehend aus H, Halogen, Alkoxy, Alkyl, fluoriertem Alkyl, teilfluoriertem Alkyl, verzweigtem oder linearem, zyklischem oder offenkettigem Alkyl, Amino, Aryl, Heteroaryl, Alkenyl, und einer elektronenziehenden Alkyl-Gruppe mit zumindest einer C=C-Doppelbindung, wobei H durch CN oder F substituiert sein kann.

2. Verbindung nach Anspruch 1, **dadurch gekennzeichnet, dass**
R1 ausgewählt ist aus der Gruppe bestehend aus Ethyl, Propyl, Butyl, verzweigtem Alkyl, und Aryl, bevorzugt aus iso-Propyl, iso-Butyl, sec-Butyl, iso-Pentyl, tert-Butyl und Phenyl; und
wobei Z1 und Z2 jeweils unabhängig voneinander O oder S sind, bevorzugt ist Z2 O.

3. Verbindung nach einem der Ansprüche 1 oder 2, **dadurch gekennzeichnet, dass** die Verbindung eine Verbindung der allgemeinen Formel II ist, 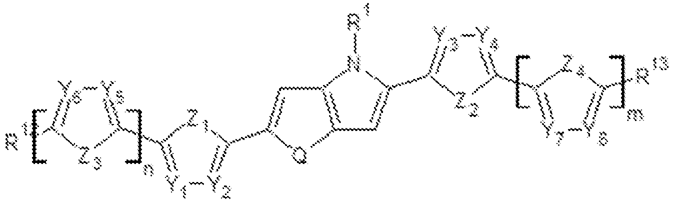 II
wobei n 0, 1 oder 2 ist und m 0, 1 oder 2 ist;
Q O oder S ist, bevorzugt ist Q S;
Z3 und Z4 jeweils unabhängig voneinander ausgewählt sind aus der Gruppe bestehend aus O, S, Se, und N-R11, wobei R11 ausgewählt ist aus der Gruppe bestehend aus H, Alkoxy, Alkyl, fluoriertem Alkyl, teilfluoriertem Alkyl, und Aryl;
R12 ausgewählt ist aus der Gruppe bestehend aus H, Alkoxy, Alkyl, fluoriertem Alkyl, teilfluoriertem Alkyl, Alkenyl, und einer elektronenziehenden Alkyl-Gruppe mit zumindest einer C=C-Doppelbindung, wobei H durch CN oder F substituiert sein kann;
R13 ausgewählt ist aus der Gruppe bestehend aus H, Alkoxy, Alkyl, fluoriertem Alkyl, teilfluoriertem Alkyl, Alkenyl, und einer elektronenziehenden Alkyl-Gruppe mit zumindest einer C=C-Doppelbindung, wobei H durch CN oder F substituiert sein kann; wobei bevorzugt Z3 und Z4 unabhängig voneinander O oder S sind;
Y5 N oder C-R14 ist, wobei R14 ausgewählt ist aus der Gruppe bestehend aus H, Halogen, Alkoxy, Alkyl, und Alkenyl, wobei H jeweils substituiert sein kann, wobei Y5 bevorzugt N, CH oder CF ist;
Y6 N oder C-R15 ist, wobei R15 ausgewählt ist aus der Gruppe bestehend aus H, Halogen, Alkoxy, Alkyl, und Alkenyl, wobei H jeweils substituiert sein kann, wobei Y6 bevorzugt N, CH oder CF ist;
Y7 N oder C-R16 ist, wobei R16 ausgewählt ist aus der Gruppe bestehend aus H, Halogen, Alkoxy, Alkyl, und Alkenyl, wobei H jeweils substituiert sein kann, wobei Y7 bevorzugt N, CH oder CF ist;
Y8 N oder C-R17 ist, wobei R17 ausgewählt ist aus der Gruppe bestehend aus H, Halogen, Alkoxy, Alkyl, und Alkenyl, wobei H jeweils substituiert sein kann, wobei Y8 bevorzugt N, CH oder CF ist; und
wobei jeweils zusammen R14 und R15, und/oder R16 und R17 einen homocyclischen Fünf-Ring oder Sechs-Ring, oder einen heterocyclischen Fünf-Ring oder Sechs-Ring mit mindestens einem Heteroatom ausgewählt aus der Gruppe bestehend aus S, O und N bilden können.

4. Verbindung nach einem der vorhergehenden Ansprüche, **dadurch gekennzeichnet, dass**
A1 eine Gruppe mit der Formel IIa 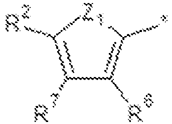 ist,
wobei R6 ausgewählt ist aus der Gruppe bestehend aus H, Halogen, Alkoxy, verzweigtem oder linearem, zyklischem oder offenkettigem Alkyl, Alkenyl, und Aryl, wobei H jeweils substituiert sein kann;
R7 ausgewählt ist aus der Gruppe bestehend aus H, Halogen, Alkoxy, verzweigtem oder linearem, zyklischem oder offenkettigem Alkyl, Alkenyl, und Aryl, wobei H jeweils substituiert sein kann; und wobei R6 und R7 zusammen einen homocyclischen Fünf-Ring oder Sechs-Ring, oder einen heterocyclischen Fünf-Ring oder Sechs-Ring mit mindestens einem Heteroatom ausgewählt aus der Gruppe bestehend aus S, O und N bilden können;
und A2 eine Gruppe mit der Formel IIb 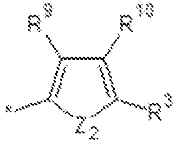 ist,
wobei R9 ausgewählt ist aus der Gruppe bestehend aus H, Halogen, Alkoxy, verzweigtem oder linearem, zyklischem oder offenkettigem Alkyl, Alkenyl, und Aryl, wobei H jeweils substituiert sein kann;
R10 ausgewählt ist aus der Gruppe bestehend aus H, Halogen, Alkoxy, verzweigtem oder linearem, zyklischem oder offenkettigem Alkyl, Alkenyl, und Aryl, wobei H jeweils substituiert sein kann; und wobei R9 und R10 zusammen einen homocyclischen Fünf-Ring oder Sechs-Ring, oder einen heterocyclischen Fünf-Ring oder Sechs-Ring mit mindestens einem Heteroatom ausgewählt aus der Gruppe bestehend aus S, O und N bilden können.

5. Verbindung nach einem der vorhergehenden Ansprüche, **dadurch gekennzeichnet, dass** die Verbindung eine Verbindung der allgemeinen Formel III, IV und/oder V ist, 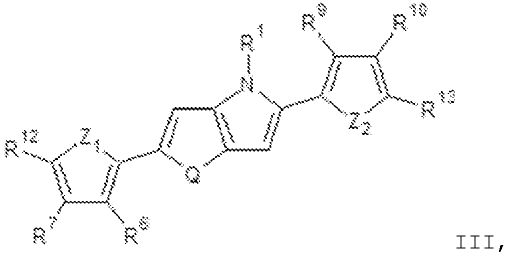 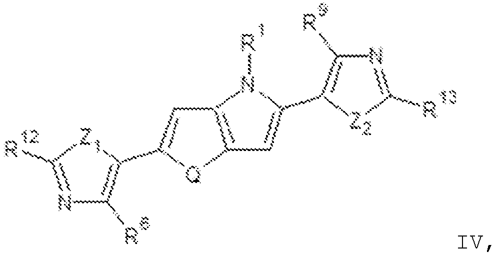 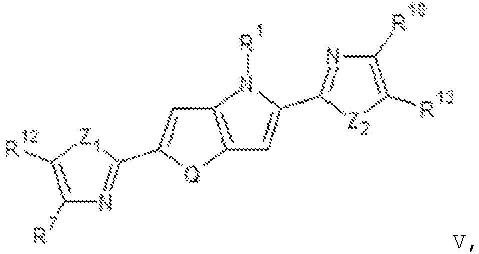
wobei Q O oder S ist, bevorzugt ist Q S;
Z1 und Z2 jeweils unabhängig voneinander O oder S sind, bevorzugt ist Z2 O;
R6 ausgewählt ist aus der Gruppe bestehend aus H, Halogen, Alkoxy, Alkyl, und Alkenyl, wobei H jeweils substituiert sein kann;
R7 ausgewählt ist aus der Gruppe bestehend aus H, Halogen, Alkoxy, Alkyl, und Alkenyl, wobei H jeweils substituiert sein kann; und
wobei R6 und R7 zusammen einen homocyclischen Fünf-Ring oder Sechs-Ring, oder einen heterocyclischen Fünf-Ring oder Sechs-Ring mit mindestens einem Heteroatom ausgewählt aus der Gruppe bestehend aus S, O und N bilden können;
R9 ausgewählt ist aus der Gruppe bestehend aus H, Halogen, Alkoxy, Alkyl, und Alkenyl, wobei H jeweils substituiert sein kann;
R10 ausgewählt ist aus der Gruppe bestehend aus H, Halogen, Alkoxy, Alkyl, und Alkenyl, wobei H jeweils substituiert sein kann; und
wobei R9 und R10 zusammen einen homocyclischen Fünf-Ring oder Sechs-Ring, oder einen heterocyclischen Fünf-Ring oder Sechs-Ring mit mindestens einem Heteroatom ausgewählt aus der Gruppe bestehend aus S, O und N bilden können;
R12 ausgewählt ist aus der Gruppe bestehend aus H, Alkoxy, Alkyl, fluoriertem Alkyl, teilfluoriertem Alkyl, Alkenyl, und einer elektronenziehenden Alkyl-Gruppe mit zumindest einer C=C-Doppelbindung, wobei H durch CN oder F substituiert sein kann; und
R13 ausgewählt ist aus der Gruppe bestehend aus H, Alkoxy, Alkyl, fluoriertem Alkyl, teilfluoriertem Alkyl, Alkenyl, und einer elektronenziehenden Alkyl-Gruppe mit zumindest einer C=C-Doppelbindung, wobei H durch CN oder F substituiert sein kann.

6. Verbindung nach einem der vorhergehenden Ansprüche, **dadurch gekennzeichnet, dass** R2 und R3, oder R12 und R13 jeweils unabhängig voneinander 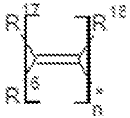 sind,
wobei n 1, 2 oder 3 ist, wobei * die Anknüpfung der Gruppe R2, R3, R12 und/oder R13 bezeichnet;
R16, R17 und R18 jeweils unabhängig voneinander ausgewählt sind aus der Gruppe bestehend aus H, Halogen, CN, COO-Alkyl, Alkyl und Alkenyl, mit der Bedingung, dass R16 und R17 nicht beide H sind, wobei bevorzugt R16 und R17 CN sind.

7. Verbindung nach einem der vorhergehenden Ansprüche, **dadurch gekennzeichnet, dass** R2 und R3, oder R12 und R13 jeweils unabhängig voneinander ausgewählt sind aus der Gruppe bestehend aus: 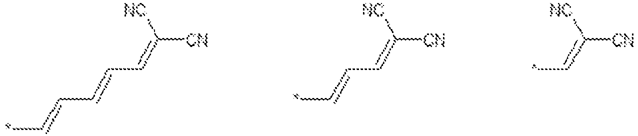 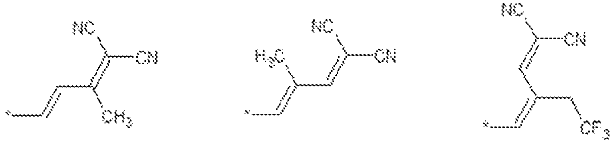 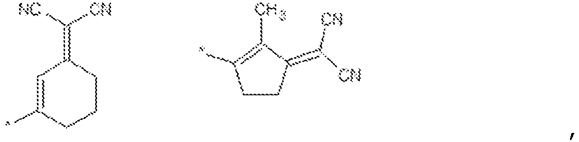
wobei * die Anknüpfung der Gruppe R2, R3, R12 und/oder R13
bezeichnet, wobei CN durch F ersetzt sein kann, und wobei bevorzugt R2 und R3, oder R12 und R13 gleich sind.

8. Verbindung nach einem der vorhergehenden Ansprüche, **dadurch gekennzeichnet, dass**
R6 und R7 H sind, R9 und R10 H sind, und Z2 O ist; wobei bevorzugt Z1 und Z2 O sind.

9. Verbindung nach einem der vorhergehenden Ansprüche, **dadurch gekennzeichnet, dass** die Verbindung ausgewählt ist aus der Gruppe bestehend aus: 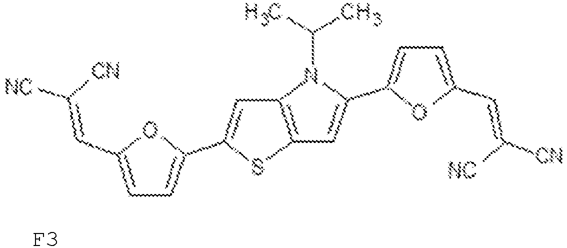 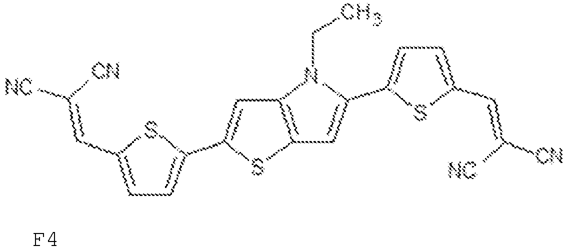 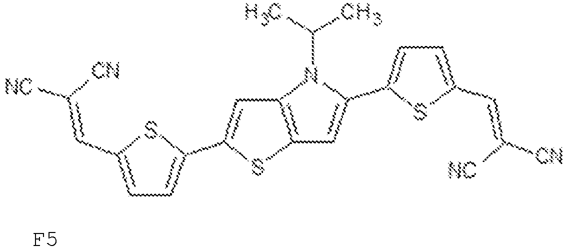 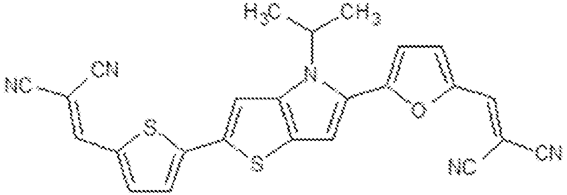 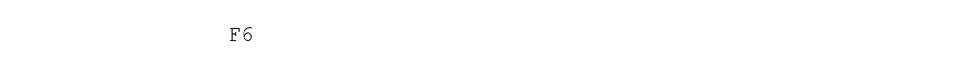 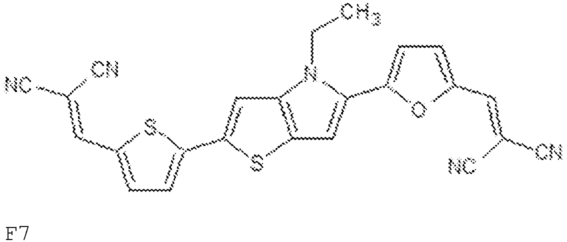 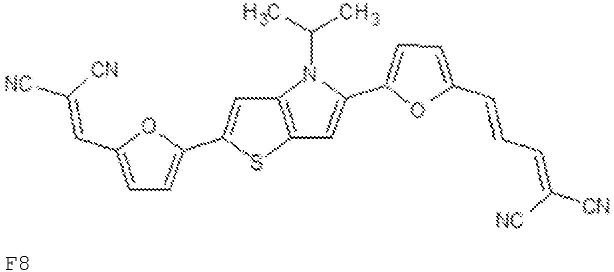 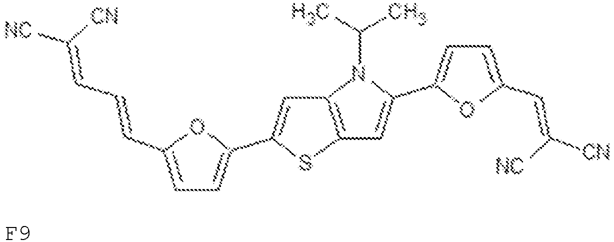 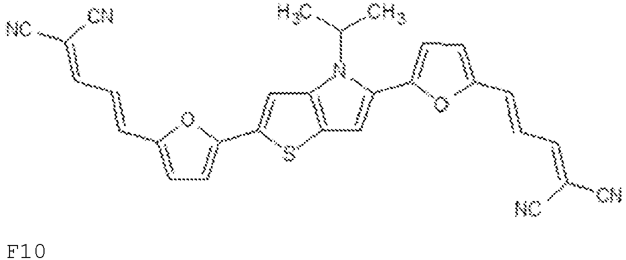 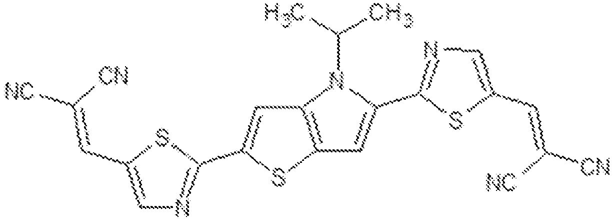 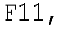 und

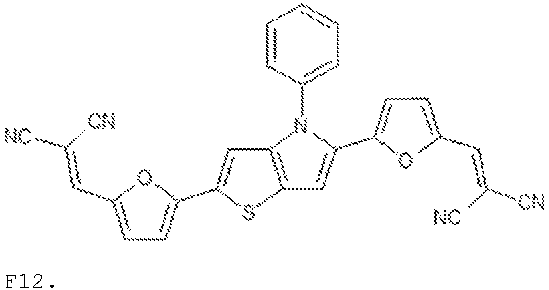

10. Verbindung nach einem der vorhergehenden Ansprüche, **dadurch gekennzeichnet, dass** die Gruppe A1 gleich der Gruppe A2 ist.

11. Optoelektronisches Bauelement, umfassend eine erste Elektrode (2), eine zweite Elektrode (6) und ein Schichtsystem (7), wobei das Schichtsystem zwischen der ersten Elektrode (2) und der zweiten Elektrode (6) angeordnet ist, **dadurch gekennzeichnet, dass** mindestens eine Schicht des Schichtsystems (7) mindestens eine Verbindung nach einem der Ansprüche 1 bis 10 aufweist.

12. Optoelektronisches Bauelement nach Anspruch 11, **dadurch gekennzeichnet, dass** das optoelektronische Bauelement ein organisches optoelektronisches Bauelement ist, bevorzugt eine organische Solarzelle, ein OFET, eine OLED oder ein organischer Photodetektor ist.

13. Optoelektronisches Bauelement nach Anspruch 11 oder 12, **dadurch gekennzeichnet, dass** das Schichtsystem (7) mindestens eine photoaktive Schicht (4) aufweist, bevorzugt eine Absorberschicht, wobei die mindestens eine photoaktive Schicht (4) die mindestens eine Verbindung nach einem der Ansprüche 1 bis 10 aufweist, bevorzugt weist das Schichtsystem (7) mindestens zwei photoaktive Schichten auf, bevorzugt mindestens drei photoaktive Schichten, oder bevorzugt mindestens vier photoaktive Schichten.

14. Optoelektronisches Bauelement nach einem der Ansprüche 11 bis 13, **dadurch gekennzeichnet, dass** die photoaktive Schicht (4) als Mischschicht aus der mindestens einen Verbindung nach einem der Ansprüche 1 bis 10 und mindestens einer weiteren Verbindung, oder als Mischschicht der mindestens einen Verbindung nach einem der Ansprüche 1 bis 10 und mindestens zwei weiteren Verbindungen ausgebildet ist, wobei die Verbindungen Absorbermaterialien sind.

15. Verwendung mindestens einer Verbindung nach einem der Ansprüche 1 bis 10 in einem organischen optoelektronischen Bauelement, wobei das organische optoelektronische Bauelement bevorzugt eine organische Solarzelle ist.

## Claims

1. Compound of general formula I 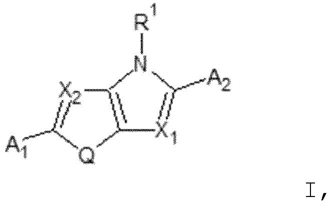 **characterized in that**
Q is O or S, preferably Q is S;
R1 is selected from the group consisting of alkoxy, alkyl, fluorinated alkyl, partially fluorinated alkyl, and aryl;
X1 and X2 are independently of one another N or C-R4; where R4 is selected from the group consisting of H, halogen, alkoxy, branched or linear, cyclic or open-chain alkyl, alkenyl, and aryl, wherein H may in each case be substituted;
A1 is a group having the formula Ia 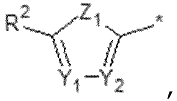
where * denotes the point of attachment to the compound of the general formula I;
Z1 is selected from the group consisting of O, S, Se, and N-R5, where R5 is selected from the group consisting of H, alkoxy, alkyl, fluorinated alkyl, partially fluorinated alkyl, and aryl;
Y1 is N or C-R6, where R6 is selected from the group consisting of H, halogen, alkoxy, branched or linear, cyclic or open-chain alkyl, alkenyl, and aryl, wherein H may in each case be substituted;
Y2 is N or C-R7, where R7 is selected from the group consisting of H, halogen, alkoxy, branched or linear, cyclic or open-chain alkyl, alkenyl, and aryl, wherein H may in each case be substituted; and where R6 and R7 may be homocyclically or heterocyclically linked to one another in the form of a ring structure;
R2 is selected from the group consisting of H, halogen, alkoxy, alkyl, fluorinated alkyl, partially fluorinated alkyl, branched or linear, cyclic or open-chain alkyl, amino, aryl, heteroaryl, alkenyl, and an electron-withdrawing alkyl group having at least one C=C double bond, wherein H may be substituted by CN or F;
A2 is a group having the formula Ib 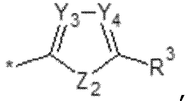
where * denotes the point of attachment to the compound of the general formula I;
Z2 is selected from the group consisting of O, S, Se, and N-R8, where R8 is selected from the group consisting of H, alkoxy, alkyl, fluorinated alkyl, partially fluorinated alkyl, and aryl;
Y3 is N or C-R9, where R9 is selected from the group consisting of H, halogen, alkoxy, branched or linear, cyclic or open-chain alkyl, alkenyl, and aryl, wherein H may in each case be substituted;
Y4 is N or C-R10, where R10 is selected from the group consisting of H, halogen, alkoxy, branched or linear, cyclic or open-chain alkyl, alkenyl, and aryl, wherein H may in each case be substituted; and where R9 and R10 may be homocyclically or heterocyclically linked to one another in the form of a ring structure; and
R3 is selected from the group consisting of H, halogen, alkoxy, alkyl, fluorinated alkyl, partially fluorinated alkyl, branched or linear, cyclic or open-chain alkyl, amino, aryl, heteroaryl, alkenyl, and an electron-withdrawing alkyl group having at least one C=C double bond, wherein H may be substituted by CN or F.

2. Compound according to Claim 1, **characterized in that**
R1 is selected from the group consisting of ethyl, propyl, butyl, branched alkyl, and aryl, preferably from isopropyl, isobutyl, sec-butyl, isopentyl, tert-butyl and phenyl; and
where Z1 and Z2 are each independently of one another O or S, preferably Z2 is O.

3. Compound according to either of Claims 1 and 2, **characterized in that** the compound is a compound of the general formula II, 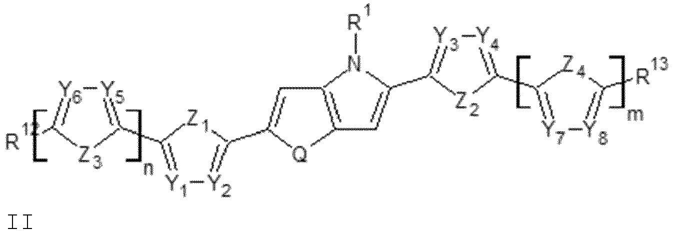
where n is 0, 1 or 2 and m is 0, 1 or 2;
Q is O or S, preferably Q is S;
Z3 and Z4 are each independently of one another selected from the group consisting of O, S, Se, and N-R11, where R11 is selected from the group consisting of H, alkoxy, alkyl, fluorinated alkyl, partially fluorinated alkyl, and aryl;
R12 is selected from the group consisting of H, alkoxy, alkyl, fluorinated alkyl, partially fluorinated alkyl, alkenyl, and an electron-withdrawing alkyl group having at least one C=C double bond, wherein H may be substituted by CN or F;
R13 is selected from the group consisting of H, alkoxy, alkyl, fluorinated alkyl, partially fluorinated alkyl, alkenyl, and an electron-withdrawing alkyl group having at least one C=C double bond, wherein H may be substituted by CN or F; where Z3 and Z4 are preferably independently of one another O or S;
Y5 is N or C-R14, where R14 is selected from the group consisting of H, halogen, alkoxy, alkyl, and alkenyl, wherein H may in each case be substituted and where Y5 is preferably N, CH or CF;
Y6 is N or C-R15, where R15 is selected from the group consisting of H, halogen, alkoxy, alkyl, and alkenyl, wherein H may in each case be substituted and where Y6 is preferably N, CH or CF;
Y7 is N or C-R16, where R16 is selected from the group consisting of H, halogen, alkoxy, alkyl, and alkenyl, wherein H may in each case be substituted and where Y7 is preferably N, CH or CF;
Y8 is N or C-R17, where R17 is selected from the group consisting of H, halogen, alkoxy, alkyl, and alkenyl, wherein H may in each case be substituted and where Y8 is preferably N, CH or CF; and
where respectively R14 and R15, and/or R16 and R17, may together form a homocyclic five-membered ring or six-membered ring, or a heterocyclic five-membered ring or six-membered ring containing at least one heteroatom selected from the group consisting of S, O, and N.

4. Compound according to any of the preceding claims, **characterized in that**
A1 is a group having the formula IIa 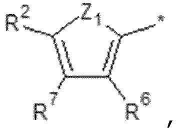
where R6 is selected from the group consisting of H, halogen, alkoxy, branched or linear, cyclic or open-chain alkyl, alkenyl, and aryl, wherein H may in each case be substituted;
R7 is selected from the group consisting of H, halogen, alkoxy, branched or linear, cyclic or open-chain alkyl, alkenyl, and aryl, wherein H may in each case be substituted; and where R6 and R7 may together form a homocyclic five-membered ring or six-membered ring, or a heterocyclic five-membered ring or six-membered ring containing at least one heteroatom selected from the group consisting of S, O and N;
and A2 is a group having the formula IIb 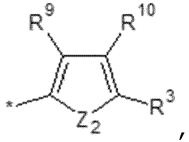
where R9 is selected from the group consisting of H, halogen, alkoxy, branched or linear, cyclic or open-chain alkyl, alkenyl, and aryl, wherein H may in each case be substituted;
R10 is selected from the group consisting of H, halogen, alkoxy, branched or linear, cyclic or open-chain alkyl, alkenyl, and aryl, wherein H may in each case be substituted; and where R9 and R10 may together form a homocyclic five-membered ring or six-membered ring, or a heterocyclic five-membered ring or six-membered ring containing at least one heteroatom selected from the group consisting of S, O and N.

5. Compound according to any of the preceding claims, **characterized in that** the compound is a compound of the general formula III, IV and/or V, 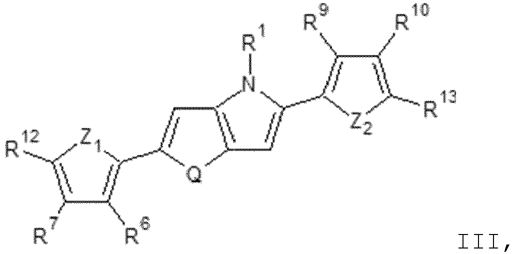 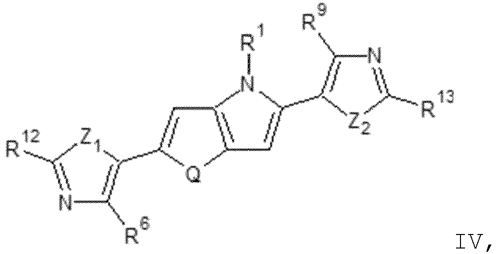 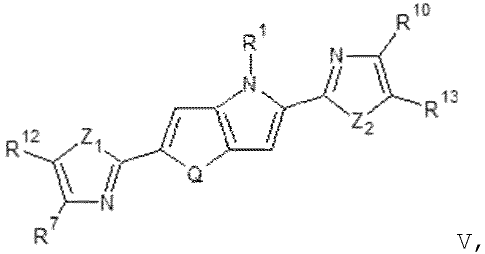
where Q is O or S, preferably Q is S;
Z1 and Z2 are each independently of one another O or S, preferably Z2 is O;
R6 is selected from the group consisting of H, halogen, alkoxy, alkyl, and alkenyl, wherein H may in each case be substituted;
R7 is selected from the group consisting of H, halogen, alkoxy, alkyl, and alkenyl, wherein H may in each case be substituted; and where R6 and R7 may together form a homocyclic five-membered ring or six-membered ring, or a heterocyclic five-membered ring or six-membered ring containing at least one heteroatom selected from the group consisting of S, O and N;
R9 is selected from the group consisting of H, halogen, alkoxy, alkyl, and alkenyl, wherein H may in each case be substituted;
R10 is selected from the group consisting of H, halogen, alkoxy, alkyl, and alkenyl, wherein H may in each case be substituted; and where R9 and R10 may together form a homocyclic five-membered ring or six-membered ring, or a heterocyclic five-membered ring or six-membered ring containing at least one heteroatom selected from the group consisting of S, O and N;
R12 is selected from the group consisting of H, alkoxy, alkyl, fluorinated alkyl, partially fluorinated alkyl, alkenyl, and an electron-withdrawing alkyl group having at least one C=C double bond, wherein H may be substituted by CN or F; and
R13 is selected from the group consisting of H, alkoxy, alkyl, fluorinated alkyl, partially fluorinated alkyl, alkenyl, and an electron-withdrawing alkyl group having at least one C=C double bond, wherein H may be substituted by CN or F.

6. Compound according to any of the preceding claims, **characterized in that** R2 and R3, or R12 and R13, are each independently of one another 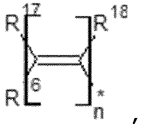
where n is 1, 2 or 3, where * denotes the point of attachment of the group R2, R3, R12 and/or R13;
R16, R17, and R18 are each independently of one another selected from the group consisting of H, halogen, CN, COO-alkyl, alkyl, and alkenyl, with the proviso that R16 and R17 are not both H, R16 and R17 preferably being CN.

7. Compound according to any of the preceding claims, **characterized in that** R2 and R3, or R12 and R13, are each independently of one another selected from the group consisting of: 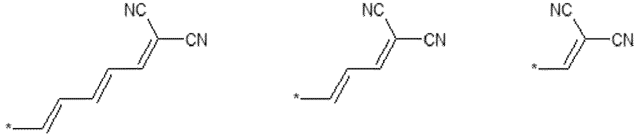 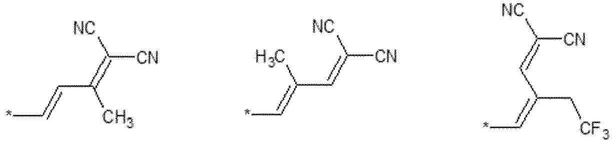 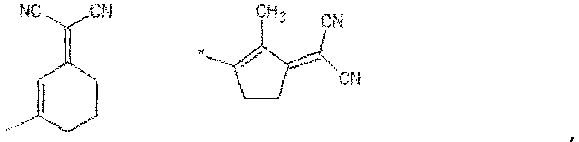 where * denotes the point of attachment of the group R2, R3, R12 and/or R13, in which CN may be replaced by F and where R2 and R3, or R12 and R13, are preferably identical.

8. Compound according to any of the preceding claims, **characterized in that**
R6 and R7 are H, R9 and R10 are H, and Z2 is O; where Z1 and Z2 are preferably O.

9. Compound according to any of the preceding claims, **characterized in that** the compound is selected from the group consisting of:

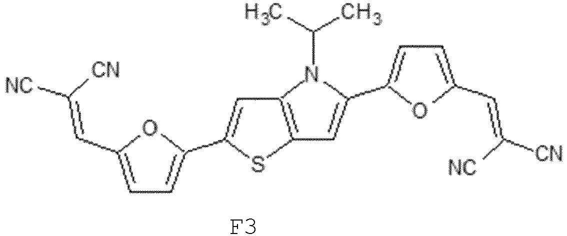

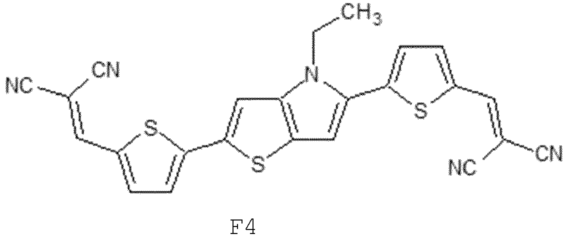

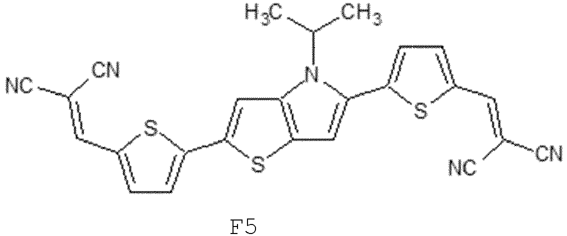

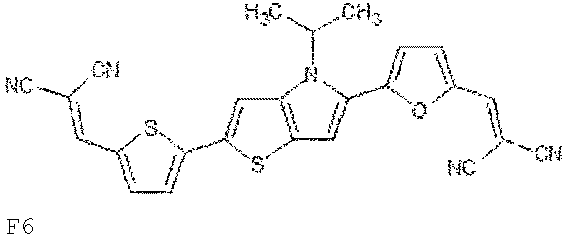

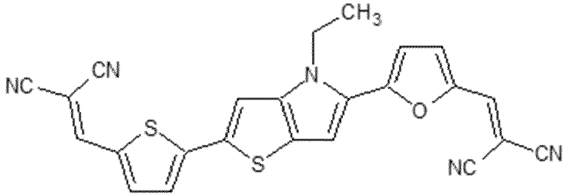

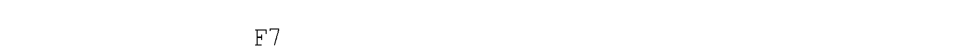

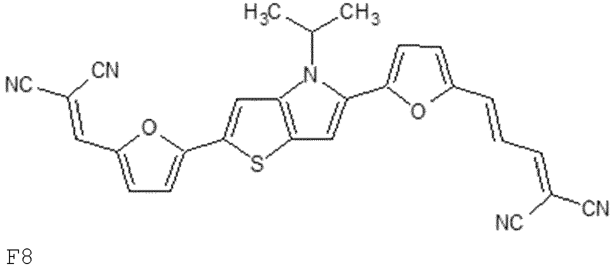

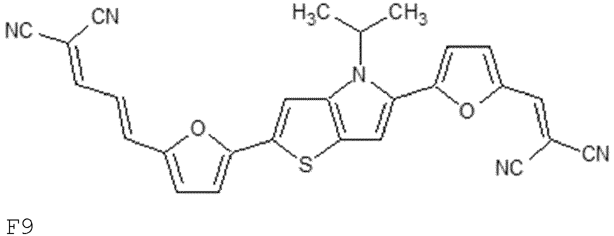

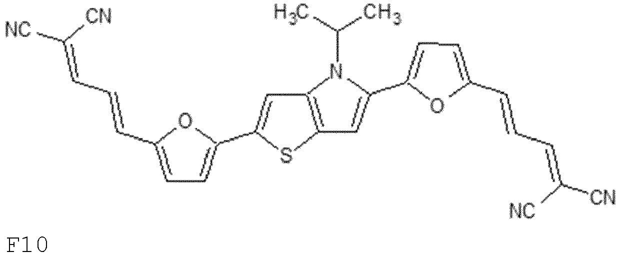

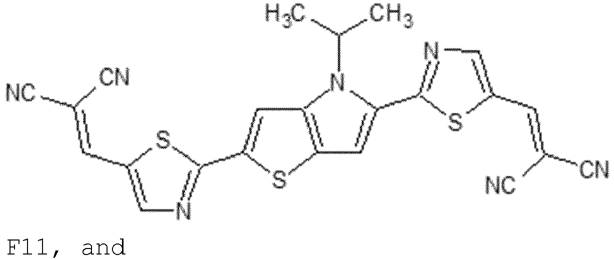

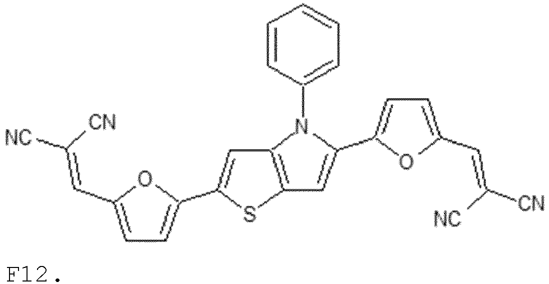

10. Compound according to any of the preceding claims, **characterized in that** group A1 is the same as group A2.

11. Optoelectronic component comprising a first electrode (2), a second electrode (6), and a layer system (7), the layer system being arranged between the first electrode (2) and the second electrode (6), **characterized in that** at least one layer of the layer system (7) comprises at least one compound according to any of Claims 1 to 10.

12. Optoelectronic component according to Claim 11, **characterized in that** the optoelectronic component is an organic optoelectronic component, preferably an organic solar cell, an OFET, an OLED or an organic photodetector.

13. Optoelectronic component according to Claim 11 or 12, **characterized in that** the layer system (7) has at least one photoactive layer (4), preferably an absorber layer, wherein the at least one photoactive layer (4) comprises the at least one compound according to any of Claims 1 to 10, preferably the layer system (7) has at least two photoactive layers, preferably at least three photoactive layers, or preferably at least four photoactive layers.

14. Optoelectronic component according to any of Claims 11 to 13, **characterized in that** the photoactive layer (4) is formed as a mixed layer of the at least one compound according to any of Claims 1 to 10 and at least one further compound, or as a mixed layer of the at least one compound according to any of Claims 1 to 10 and at least two further compounds, the compounds being absorber materials.

15. Use of at least one compound according to any of Claims 1 to 10 in an organic optoelectronic component, the organic optoelectronic component preferably being an organic solar cell.

## Revendications

1. Composé de formule générale I 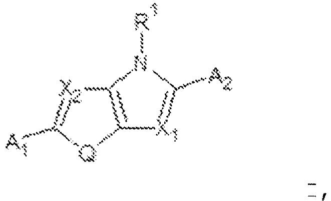 **caractérisé en ce que**
Q représente O ou S ; Q représentant de préférence S ;
R¹ est choisi dans le groupe constitué par alcoxy, alkyle, alkyle fluoré, alkyle partiellement fluoré et aryle;
X₁ et X₂ représentent, indépendamment l'un de l'autre, N ou C-R⁴;
R⁴ étant choisi dans le groupe constitué par H, halogène, alcoxy, alkyle ramifié ou linéaire, cyclique ou à chaîne ouverte, alcényle et aryle, H pouvant à chaque fois être substitué;
A₁ représente un groupe de formule Ia 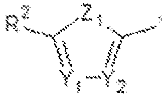
dans laquelle
* désigne la liaison au composé de formule générale I;
Z₁ est choisi dans le groupe constitué par O, S, Se, et N-R⁵, R⁵ étant choisi dans le groupe constitué par H, alcoxy, alkyle, alkyle fluoré, alkyle partiellement fluoré et aryle;
Y₂ représente N ou C-R⁶, R⁶ étant choisi dans le groupe constitué par H, halogène, alcoxy, alkyle ramifié ou linéaire, cyclique ou à chaîne ouverte, alcényle et aryle, H pouvant à chaque fois être substitué;
Y₂ représente N ou C-R⁷, R⁷ étant choisi dans le groupe constitué par H, halogène, alcoxy, alkyle ramifié ou linéaire, cyclique ou à chaîne ouverte, alcényle et aryle, H pouvant à chaque fois être substitué ; et R⁶ et R⁷ pouvant être liés l'un à l'autre de manière homocyclique ou hétérocyclique sous forme d'une structure cyclique:
R² est choisi dans le groupe constitué par H, halogène, alcoxy, alkyle, alkyle fluoré, alkyle partiellement fluoré, alkyle ramifié ou linéaire, cyclique ou à chaîne ouverte, amino, aryle, hétéroaryle, alcényle et un groupe alkyle attracteur d'électrons présentant au moins une double liaison C=C, H pouvant être substitué par CN ou F ;
A₂ représente un groupe de formule Ib 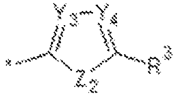
dans laquelle
* désigne la liaison au composé de formule générale I;
Z₂ est choisi dans le groupe constitué par O, S, Se, et N-R⁸, R⁸ étant choisi dans le groupe constitué par H, alcoxy, alkyle, alkyle fluoré, alkyle partiellement fluoré et aryle;
Y₃ représente N ou C-R⁹, R⁹ étant choisi dans le groupe constitué par H, halogène, alcoxy, alkyle ramifié ou linéaire, cyclique ou à chaîne ouverte, alcényle et aryle, H pouvant à chaque fois être substitué;
Y₄ représente N ou C-R¹⁰, R¹⁰ étant choisi dans le groupe constitué par H, halogène, alcoxy, alkyle ramifié ou linéaire, cyclique ou à chaîne ouverte, alcényle et aryle, H pouvant à chaque fois être substitué ; et R⁹ et R¹⁰ pouvant être liés l'un à l'autre de manière homocyclique ou hétérocyclique sous forme d'une structure cyclique:
R³ est choisi dans le groupe constitué par H, halogène, alcoxy, alkyle, alkyle fluoré, alkyle partiellement fluoré, alkyle ramifié ou linéaire, cyclique ou à chaîne ouverte, amino, aryle, hétéroaryle, alcényle et un groupe alkyle attracteur d'électrons présentant au moins une double liaison C=C, H pouvant être substitué par CN ou F.

2. Composé selon la revendication 1, **caractérisé en ce que**
R¹ est choisi dans le groupe constitué par éthyle, propyle, butyle, alkyle ramifié et aryle, de préférence parmi iso-propyle, iso-butyle, secbutyle, iso-pentyle, tert-butyle et phényle ; et où
Z₁ et Z₂ représentent, à chaque fois indépendamment l'un de l'autre, O ou S ; Z₂ représentant de préférence O.

3. Composé selon l'une quelconque des revendications 1 ou 2, **caractérisé en ce que** le composé est un composé de formule générale II, 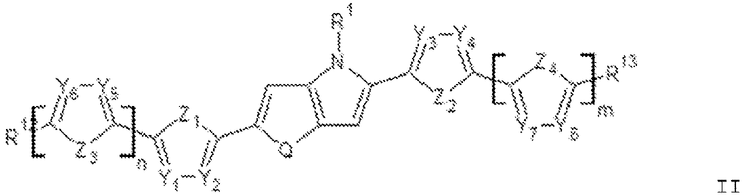 dans laquelle
n vaut 0, 1 ou 2 et m vaut 0, 1 ou 2 ;
Q représente O ou S ; Q représentant de préférence S ;
Z₃ et Z₄ sont choisis, à chaque fois indépendamment l'un de l'autre, dans le groupe constitué par O, S, Se et N-R¹¹, R¹¹ étant choisi dans le groupe constitué par H, alcoxy, alkyle, alkyle fluoré, alkyle partiellement fluoré et aryle;
R¹² est choisi dans le groupe constitué par H, alcoxy, alkyle, alkyle fluoré, alkyle partiellement fluoré, alcényle et un groupe alkyle attracteur d'électrons présentant au moins une double liaison C=C, H pouvant être substitué par CN ou F;
R¹³ est choisi dans le groupe constitué par H, alcoxy, alkyle, alkyle fluoré, alkyle partiellement fluoré, alcényle et un groupe alkyle attracteur d'électrons présentant au moins une double liaison C=C, H pouvant être substitué par CN ou F ; Z₃ et Z₄ représentant de préférence, indépendamment l'un de l'autre, O ou S;
Y₅ représente N ou C-R¹⁴, R¹⁴ étant choisi dans le groupe constitué par H, halogène, alcoxy, alkyle et alcényle, H pouvant à chaque fois être substitué, Y₅ représentant de préférence N, CH ou CF;
Y₆ représente N ou C-R¹⁵, R¹⁵ étant choisi dans le groupe constitué par H, halogène, alcoxy, alkyle et alcényle, H pouvant à chaque fois être substitué, Y₆ représentant de préférence N, CH ou CF;
Y₇ représente N ou C-R¹⁶, R¹⁶ étant choisi dans le groupe constitué par H, halogène, alcoxy, alkyle et alcényle, H pouvant à chaque fois être substitué, Y₇ représentant de préférence N, CH ou CF;
Y₈ représente N ou C-R¹⁷, R¹⁷ étant choisi dans le groupe constitué par H, halogène, alcoxy, alkyle et alcényle, H pouvant à chaque fois être substitué, Y₈ représentant de préférence N, CH ou CF; et
où R¹⁴ et R¹⁵ et/ou R¹⁶ et R¹⁷ peuvent former à chaque fois, conjointement, un cycle homocyclique à cinq ou six chaînons ou un cycle hétérocyclique à cinq ou six chaînons, comprenant au moins un hétéroatome choisi dans le groupe constitué par S, O et N.

4. Composé selon l'une quelconque des revendications précédentes, **caractérisé en ce que**
A₁ représente un groupe de formule IIa 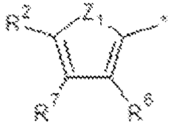
dans laquelle
R⁶ est choisi dans le groupe constitué par H, halogène, alcoxy, alkyle ramifié ou linéaire, cyclique ou à chaîne ouverte, alcényle et aryle, H pouvant à chaque fois être substitué;
R⁷ est choisi dans le groupe constitué par H, halogène, alcoxy, alkyle ramifié ou linéaire, cyclique ou à chaîne ouverte, alcényle et aryle, H pouvant à chaque fois être substitué; et où R⁶ et R⁷ peuvent former conjointement, un cycle homocyclique à cinq ou six chaînons ou un cycle hétérocyclique à cinq ou six chaînons, comprenant au moins un hétéroatome choisi dans le groupe constitué par S, O et N ; et
A₂ représente un groupe de formule IIb 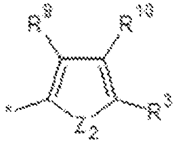
dans laquelle
R⁹ est choisi dans le groupe constitué par H, halogène, alcoxy, alkyle ramifié ou linéaire, cyclique ou à chaîne ouverte, alcényle et aryle, H pouvant à chaque fois être substitué;
R¹⁰ est choisi dans le groupe constitué par H, halogène, alcoxy, alkyle ramifié ou linéaire, cyclique ou à chaîne ouverte, alcényle et aryle, H pouvant à chaque fois être substitué; et où R⁹ et R¹⁰ peuvent former conjointement, un cycle homocyclique à cinq ou six chaînons ou un cycle hétérocyclique à cinq ou six chaînons, comprenant au moins un hétéroatome choisi dans le groupe constitué par S, O et N.

5. Composé selon l'une quelconque des revendications précédentes, **caractérisé en ce que** le composé est un composé de formule générale III, IV et/ou V, 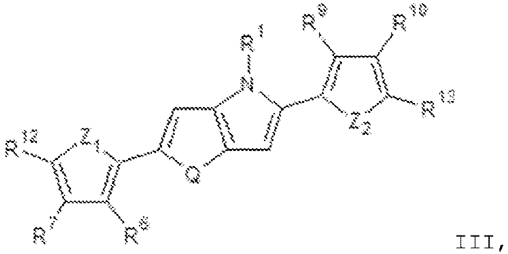 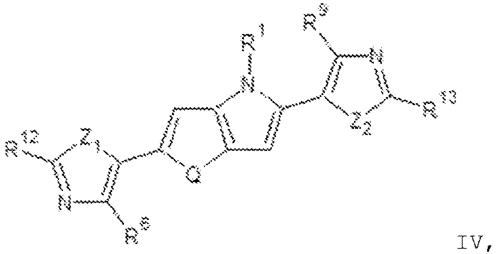 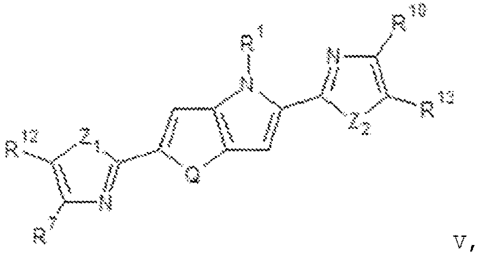 dans lesquelles
Q représente O ou S, Q représentant de préférence S;
Z₁ et Z₂ représentent, à chaque fois indépendamment l'un de l'autre, O ou S, Z₂ représentant de préférence O;
R⁶ est choisi dans le groupe constitué par H, halogène, alcoxy, alkyle et alcényle, H pouvant à chaque fois être substitué;
R⁷ est choisi dans le groupe constitué par H, halogène, alcoxy, alkyle et alcényle, H pouvant à chaque fois être substitué; et où R⁶ et R⁷ peuvent former conjointement, un cycle homocyclique à cinq ou six chaînons ou un cycle hétérocyclique à cinq ou six chaînons, comprenant au moins un hétéroatome choisi dans le groupe constitué par S, O et N;
R⁹ est choisi dans le groupe constitué par H, halogène, alcoxy, alkyle et alcényle, H pouvant à chaque fois être substitué;
R¹⁰ est choisi dans le groupe constitué par H, halogène, alcoxy, alkyle et alcényle, H pouvant à chaque fois être substitué; et où R⁹ et R¹⁰ peuvent former conjointement, un cycle homocyclique à cinq ou six chaînons ou un cycle hétérocyclique à cinq ou six chaînons, comprenant au moins un hétéroatome choisi dans le groupe constitué par S, O et N;
R¹² est choisi dans le groupe constitué par H, alcoxy, alkyle, alkyle fluoré, alkyle partiellement fluoré, alcényle et un groupe alkyle attracteur d'électrons présentant au moins une double liaison C=C, H pouvant être substitué par CN ou F; et
R¹³ est choisi dans le groupe constitué par H, alcoxy, alkyle, alkyle fluoré, alkyle partiellement fluoré, alcényle et un groupe alkyle attracteur d'électrons présentant au moins une double liaison C=C, H pouvant être substitué par CN ou F.

6. Composé selon l'une quelconque des revendications précédentes, **caractérisé en ce que** R² et R3, ou R¹² et R¹³ représentent, à chaque fois indépendamment l'un de l'autre 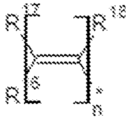 dans laquelle
n vaut 1, 2 ou 3, où
* désigne la liaison des groupes R², R3, R¹² et/ou R¹³;
R¹⁶, R¹⁷ et R¹⁸ sont choisis, à chaque fois indépendamment les uns des autres, dans le groupe constitué par H, halogène, CN, COO-alkyle, alkyle et alcényle, à condition que R¹⁶ et R¹⁷ ne représentent pas tous les deux H, R¹⁶ et R¹⁷ représentant de préférence CN.

7. Composé selon l'une quelconque des revendications précédentes, **caractérisé en ce que** R² et R³ ou R¹² et R¹³ sont choisis, à chaque fois indépendamment les uns des autres, dans le groupe constitué par 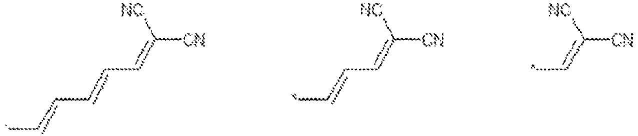 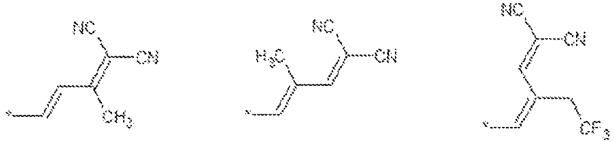 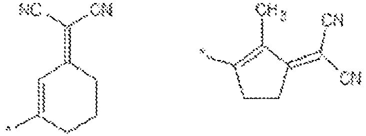 où * désigne la liaison des groupes R², R³, R¹² et/ou R¹³, où CN peut être remplacé par F et où, de préférence, R² et R³, ou R¹² et R¹³ sont identiques.

8. Composé selon l'une quelconque des revendications précédentes, **caractérisé en ce que** R⁶ et R⁷ représentent H; R⁹ et R¹⁰ représentent H; et Z₂ représente O; Z₁ et Z₂ représentant de préférence O.

9. Composé selon l'une quelconque des revendications précédentes, **caractérisé en ce que** le composé est choisi dans le groupe constitué par :

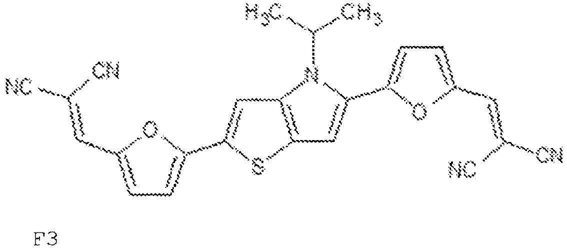

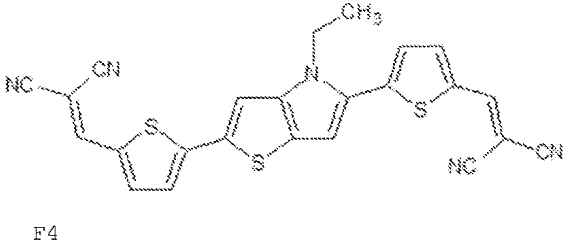

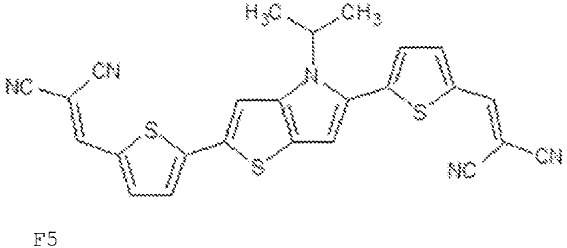

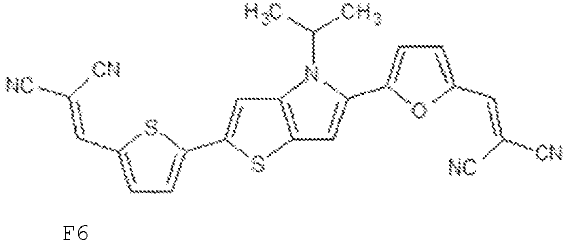

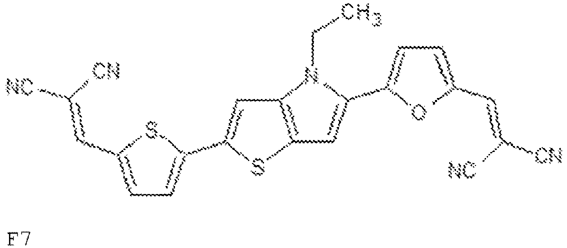

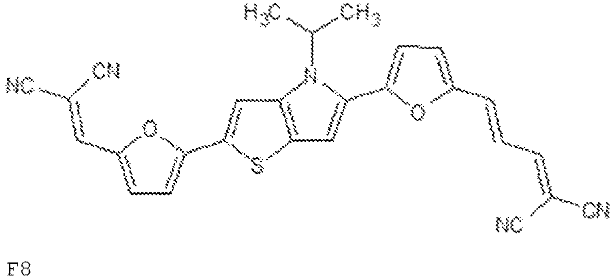

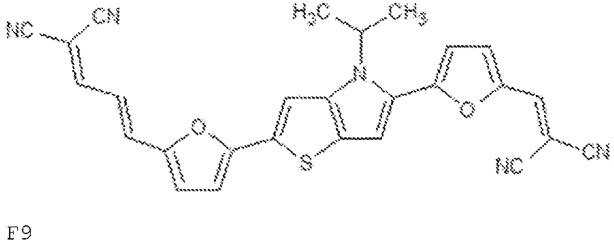

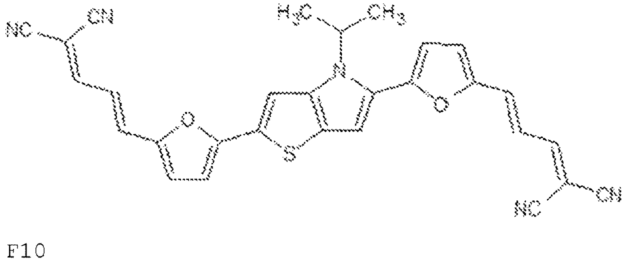

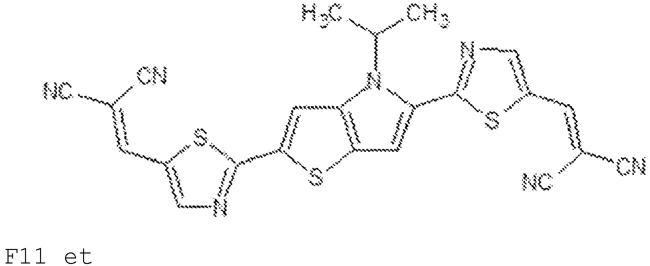

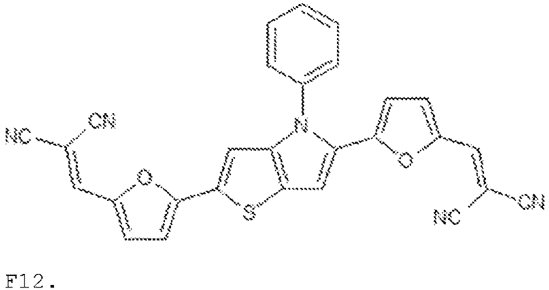

10. Composé selon l'une quelconque des revendications précédentes, **caractérisé en ce que** le groupe A₁ est identique au groupe A₂.

11. Composant optoélectronique, comprenant une première électrode (2), une deuxième électrode (6) et un système stratifié (7), le système stratifié étant agencé entre la première électrode (2) et la deuxième électrode (6), **caractérisé**
**en ce qu'**au moins une couche du système stratifié (7) présente au moins un composé selon l'une quelconque des revendications 1 à 10.

12. Composant optoélectronique selon la revendication 11, **caractérisé en ce que** le composé optoélectronique est un composant optoélectronique organique, de préférence une cellule solaire organique, un transistor à effet de champ organique (TEFO), une DELO ou un photodétecteur organique.

13. Composant optoélectronique selon la revendication 11 ou 12, **caractérisé en ce que** le système stratifié (7) présente au moins une couche photoactive (4), de préférence une couche absorbante, ladite au moins une couche photoactive (4) présentant ledit au moins un composé selon l'une quelconque des revendications 1 à 10 ; de préférence, le système stratifié (7) présente au moins deux couches photoactives, de préférence au moins trois couches photoactives ou de préférence au moins quatre couches photoactives.

14. Composant optoélectronique selon l'une quelconque des revendications 11 à 13, **caractérisé en ce que** la couche photoactive (4) est conçue en tant que couche mixte constituée par ledit au moins un composé selon l'une quelconque des revendications 1 à 10 et au moins un autre composé ou en tant que couche mixte dudit au moins un composé selon l'une quelconque des revendications 1 à 10 et au moins deux autres composés, les composés étant des matériaux absorbants.

15. Utilisation d'au moins un composé selon l'une quelconque des revendications 1 à 10 dans un composant optoélectronique organique, le composant optoélectronique organique étant de préférence une cellule solaire organique.
